(19) Europäisches Patentamt
European Patent Office
Office européen des brevets

(11) EP 4 616 860 A1

(12) **EUROPEAN PATENT APPLICATION**
published in accordance with Art. 153(4) EPC

(43) Date of publication:
**17.09.2025 Bulletin 2025/38**

(21) Application number: **23888115.5**

(22) Date of filing: **10.11.2023**

(51) International Patent Classification (IPC):
***A61K 31/444** (2006.01)* ***C07D 401/04** (2006.01)*
***A61P 25/00** (2006.01)*

(52) Cooperative Patent Classification (CPC):
**A61K 31/444; A61P 25/00; C07D 401/04**

(86) International application number:
**PCT/CN2023/130976**

(87) International publication number:
**WO 2024/099430 (16.05.2024 Gazette 2024/20)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **10.11.2022 CN 202211406281**

(71) Applicant: **Xizang Haisco Pharmaceutical Co., Ltd.**
**Lhoka, Tibet 856099 (CN)**

(72) Inventors:
- **ZHANG, Xuanmiao**
  **Lhoka, Tibet 856099 (CN)**
- **MAO, Hua**
  **Lhoka, Tibet 856099 (CN)**
- **SONG, Hang**
  **Lhoka, Tibet 856099 (CN)**
- **DENG, Shihao**
  **Lhoka, Tibet 856099 (CN)**
- **ZHOU, Yang**
  **Lhoka, Tibet 856099 (CN)**
- **DOU, Ying**
  **Lhoka, Tibet 856099 (CN)**

(74) Representative: **IP TRUST SERVICES**
**Europole - Le Grenat**
**3, avenue Doyen Louis Weil**
**38000 Grenoble (FR)**

(54) **PHARMACEUTICAL COMPOSITION COMPRISING AAK1 INHIBITOR**

(57) Provided are a pharmaceutical composition of a compound of formula (I) or a stereoisomer or a pharmaceutically acceptable salt thereof, and a pharmaceutical formulation thereof; and a preparation method for the pharmaceutical composition and the pharmaceutical formulation, and use of the pharmaceutical composition and the pharmaceutical formulation in the preparation of a medicament for treating and inhibiting or degrading AAK1-related diseases.

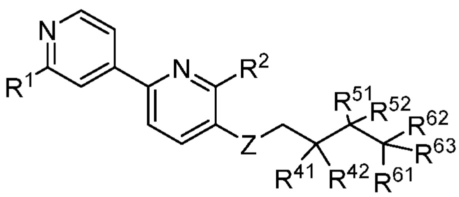

(I)

EP 4 616 860 A1

## Description

### Technical Field

The present invention relates to a pharmaceutical composition of a compound of formula (I) or a stereoisomer or a pharmaceutically acceptable salt thereof, and a pharmaceutical formulation thereof, and the use of the pharmaceutical composition and the pharmaceutical formulation in the preparation of a medicament for treating an AAK1-related disease by inhibition of an AAK1 inhibitor.

### Background Art

Neuropathic pain (NP) is a general term for a range of pains caused by damage and diseases of the somatic sensory nervous system. It is divided into peripherally-induced neuropathic pain (pNP) and central neuropathic pain. pNP is more common clinically, and can be divided into diabetic peripheral neuralgia, postherpetic neuralgia, trigeminal neuralgia, postoperative chronic neuralgia, etc. Clinically, pNP patients often have symptoms such as spontaneous pain (pain without any external stimulation), allodynia (increased response to painful stimuli), hyperalgesia (feeling pain in response to stimuli that are normally painless) and paraesthesia, which seriously affect the patients' quality of life.

AAK1 is a member of the Ark1/Prk1 family of serine/threonine kinases and is widely expressed in the brain and spinal cord. Studies have shown that AAK1 knockout mice exhibit responses to persistent pain or hypoalgesia, and AAK1 knockout mice do not develop hyperalgesia in the neuropathic pain model of spinal nerve ligation, which confirms that AAK1 is a feasible target for the treatment of pNP.

We provide an AAK1 inhibitor, but the compound has a low melting point and often adheres to the capsule punch or tablet punch when being prepared into tablets or capsules. Based on this, we intend to provide a composition containing an AAK1 inhibitor, which can be advantageously applied to the preparation of conventional solid formulations, and the resulting formulation has good in vitro dissolution behaviour and stability.

### Summary of the Invention

The present invention provides a pharmaceutical composition of a compound of formula (I) and a stereoisomer and a pharmaceutically acceptable salt thereof, and a pharmaceutical formulation thereof.

The pharmaceutical composition or the pharmaceutical formulation of the present invention has good solubility, dissolution rate, bioavailability, oral performance, stable quality, good safety and low irritation, and meets the drug quality standards.

In one aspect, the present invention provides a pharmaceutical composition, comprising:

an active ingredient, wherein the active ingredient is selected from a compound of formula (I), or a stereoisomer or a pharmaceutically acceptable salt thereof:

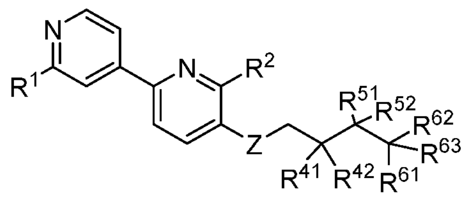

(I)

wherein

Z is selected from NH or O;

$R^1$ and $R^2$ are each independently selected from H, deuterium, halogen, amino, -COOH, cyano, sulfonyl, aminoacyl, $C_{1-6}$ alkyl, halo $C_{1-6}$ alkyl, or deuterated $C_{1-6}$ alkyl, wherein the alkyl is optionally further substituted with 1-3 $R^A$ substituents;

$R^{41}$ and $R^{42}$ are each independently selected from H, deuterium, amino, $C_{1-6}$ alkyl, halogen, cyano, hydroxyl, halo $C_{1-6}$ alkyl, or deuterated $C_{1-6}$ alkyl;

$R^{51}$ and $R^{52}$ are each independently selected from H, deuterium, amino, or halogen;

$R^{61}$, $R^{62}$ and $R^{63}$ are each independently selected from H, deuterium, halogen, amino, cyano, hydroxyl, $C_{1-6}$ alkyl, halo $C_{1-6}$ alkyl, or deuterated $C_{1-6}$ alkyl;

or, $R^{51}$ and $R^{61}$, or $R^{61}$ and $R^{62}$ together with the carbon atom(s) to which they are each attached form a double bond;

$R^A$ is selected from deuterium, halogen, amino, cyano, hydroxyl, $C_{1-6}$ alkyl, halo $C_{1-6}$ alkyl, deuterated $C_{1-6}$ alkyl, $C_{1-6}$ alkoxy, halo $C_{1-6}$ alkoxy, deuterated $C_{1-6}$ alkoxy, or hydroxy $C_{1-6}$ alkyl,
provided that when Z is selected from O,

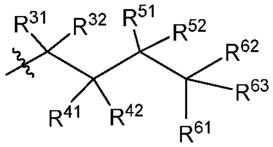

does not form the following structures:

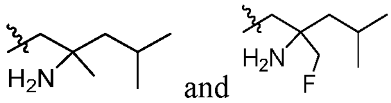

a non-active ingredient;
wherein the active ingredient is present in the pharmaceutical composition in an amount of 5% to 90% w/w, preferably 5% to 80% w/w, and more preferably 10% to 80% w/w;
in some embodiments, the active ingredient (calculated as a free base) is present in the pharmaceutical composition in an amount of 5% to 85% w/w, preferably 8% to 80% w/w, and more preferably 10% to 80% w/w;
in some embodiments, the active ingredient (calculated as a free base) is present in the pharmaceutical composition in an amount of 8% to 85% w/w, preferably 10% to 85% w/w, and more preferably 10% to 80% w/w;
in some embodiments, the active ingredient (calculated as a free base) is present in the pharmaceutical composition in an amount of 5% to 80% w/w, 5% to 70% w/w, 5% to 60% w/w, 5% to 50% w/w, 5% to 40% w/w, 5% to 30% w/w, 5% to 20% w/w, 10% to 80% w/w, 10% to 70% w/w, 10% to 60% w/w, 10% to 50% w/w, 10% to 40% w/w, 10% to 30% w/w, or 10% to 20% w/w.

In some embodiments, in the compound of formula (I), Z is O;

$R^1$ and $R^2$ are selected from halo $C_{1-2}$ alkyl;
$R^{51}$ and $R^{52}$ are each independently selected from H or deuterium;
$R^{41}$ and $R^{42}$ are each independently selected from amino or $C_{1-2}$ alkyl;
$R^{61}$, $R^{62}$ and $R^{63}$ are each independently selected from H, deuterium, $C_{1-2}$ alkyl, or halo $C_{1-2}$ alkyl;
or, $R^{51}$ and $R^{61}$, or $R^{61}$ and $R^{62}$ together with the carbon atom(s) to which they are each attached form a double bond;
in some embodiments, in the compound of formula (I),
$R^1$ and $R^2$ are each independently selected from $-CH_2F$, $-CHF_2$, $-CF_3$, $-CH_2CH_2F$, $-CH_2CHF_2$, $-CH_2CF_3$, $-CHFCH_2F$, $-CHFCHF_2$, $-CHFCF_3$, $-CF_2CH_2F$, $-CF_2CHF_2$, $-CF_2CF_3$, $-CH_2Cl$, $-CHCl_2$, $-CCl_3$, $-CH_2CH_2Cl$, $-CH_2CHCl_2$, $-CH_2CCl_3$, $-CHClCH_2Cl$, $-CHClCHCl_2$, $-CHClCCl_3$, $-CCl_2CH_2Cl$, $-CCl_2CHCl_2$, or $-CCl_2CCl_3$;
$R^{51}$ and $R^{52}$ are each independently selected from H or deuterium;
$R^{41}$ and $R^{42}$ are each independently selected from amino, $-CH_3$, or $-CH_2CH_3$;
$R^{61}$, $R^{62}$ and $R^{63}$ are each independently selected from H, deuterium, $-CH_3$, $-CH_2CH_3$, $-CH_2F$, $-CHF_2$, $-CF_3$, $-CH_2CH_2F$, $-CH_2CHF_2$, $-CH_2CF_3$, $-CHFCH_2F$, $-CHFCHF_2$, $-CHFCF_3$, $-CF_2CH_2F$, $-CF_2CHF_2$, $-CF_2CF_3$, $-CH_2Cl$, $-CHCl_2$, $-CCl_3$, $-CH_2CH_2Cl$, $-CH_2CHCl_2$, $-CH_2CCl_3$, $-CHClCH_2Cl$, $-CHClCHCl_2$, $-CHClCCl_3$, $-CCl_2CH_2Cl$, $-CCl_2CHCl_2$, or $-CCl_2CCl_3$;
or, $R^{51}$ and $R^{61}$, or $R^{61}$ and $R^{62}$ together with the carbon atom(s) to which they are each attached form a double bond;
in some embodiments, in the compound of formula (I),
Z is O;
$R^1$ and $R^2$ are each independently selected from $-CH_2F$, $-CHF_2$, or $-CF_3$;
$R^{51}$ and $R^{52}$ are each independently selected from H or deuterium;
$R^{41}$ and $R^{42}$ are each independently selected from amino or $-CH_3$;
$R^{61}$, $R^{62}$ and $R^{63}$ are each independently selected from H, deuterium, $-CH_3$, or $CF_3$;
in some embodiments, in the compound of formula (I),
Z is O;
$R^1$ and $R^2$ are each independently selected from $-CH_2F$, $-CHF_2$, or $-CF_3$;
$R^{51}$ and $R^{52}$ together with the carbon atom(s) to which they are each attached form a double bond;
$R^{41}$ and $R^{42}$ are each independently selected from amino or $-CH_3$;
$R^{61}$, $R^{62}$ and $R^{63}$ are each independently selected from H, deuterium, $-CH_3$, or $CF_3$;

in some embodiments, in the compound of formula (I),

Z is O;

$R^1$ and $R^2$ are each independently selected from $-CH_2F$, $-CHF_2$, or $-CF_3$;

$R^{51}$ and $R^{52}$ are each independently selected from H or deuterium;

$R^{41}$ and $R^{42}$ are each independently selected from amino or $-CH_3$;

each $R^{63}$ is independently selected from H, deuterium, $-CH_3$, or $CF_3$;

$R^{61}$ and $R^{62}$ together with the carbon atom(s) to which they are each attached form a double bond;

in some embodiments, the compound of formula (I) is selected from one of the following structures:

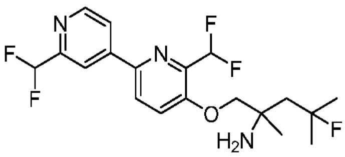
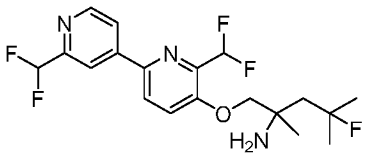
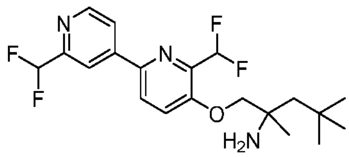

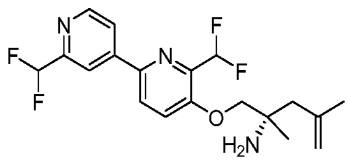
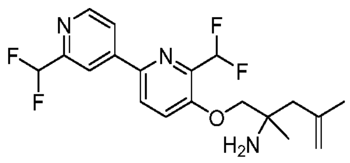
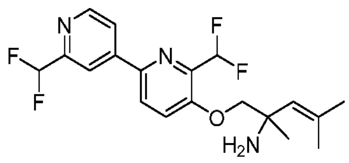

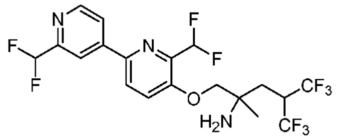
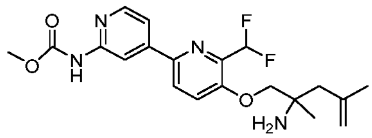
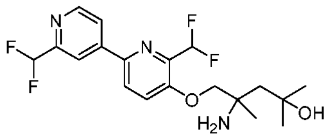

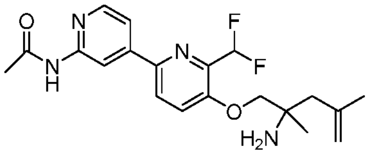
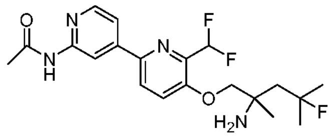
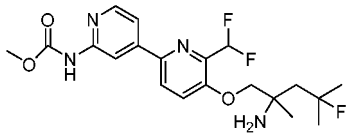

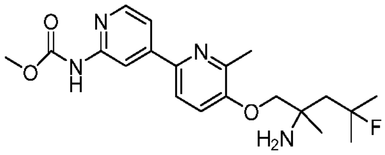
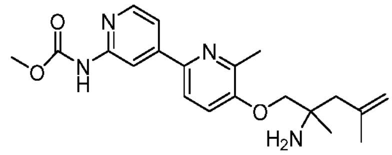

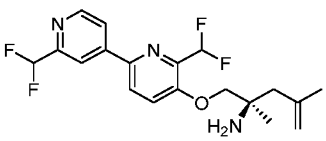
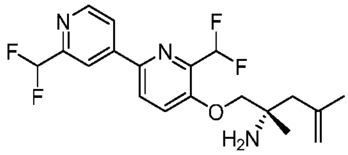
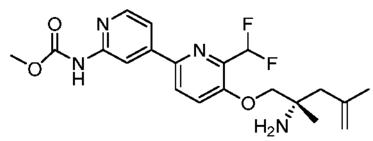

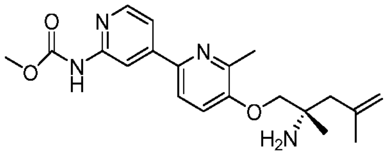
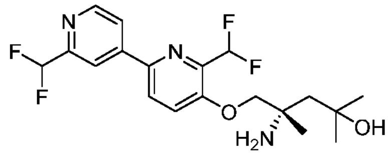

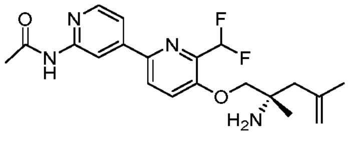
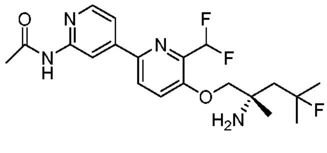
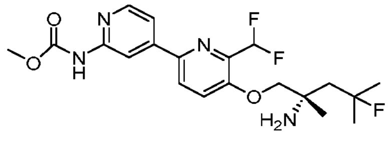

or 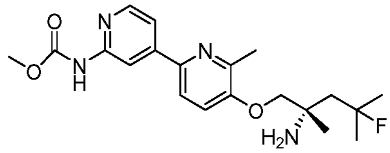;

in some embodiments, the compound of formula (I) is selected from one of the following structures:

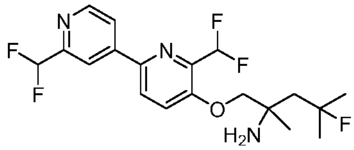 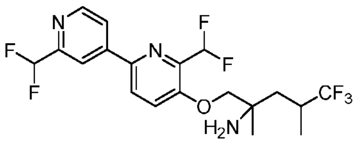 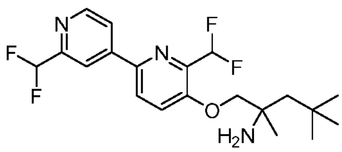

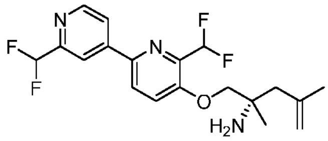 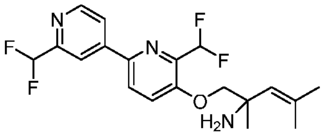 or 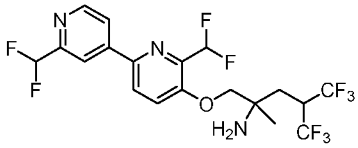;

in some embodiments, the non-active ingredient contains an infiltrating agent;
in some embodiments, the non-active ingredient contains a disintegrant;
in some embodiments, the non-active ingredient contains a diluent;
in some embodiments, the non-active ingredient contains a lubricant;
in some embodiments, the infiltrating agent is selected from one or more of silicates; preferably, the infiltrating agent is one or more of silica, magnesium silicate, magnesium trisilicate, magnesium aluminium silicate and talc; more preferably, the infiltrating agent is one or more of fumed silica, precipitated silica, sol-gel silica, magnesium silicate, magnesium trisilicate, magnesium aluminium silicate and talc;
in some embodiments, the disintegrant is selected from one or more of croscarmellose sodium, crospovidone, starch and a derivative thereof, low-substituted hydroxypropyl cellulose, low-substituted sodium hydroxymethyl cellulose, a surfactant, alginic acid and sodium alginate, and clays; preferably, the disintegrant is one or more of croscarmellose sodium, low-substituted hydroxypropyl cellulose, low-substituted sodium hydroxymethyl cellulose, crospovidone, starch, sodium carboxymethyl starch, hydroxypropyl starch, polysorbate 80, sodium lauryl sulphate, bentonite, and colloidal magnesium aluminium silicate; more preferably, the disintegrant is one or more of croscarmellose sodium, low-substituted hydroxypropyl cellulose, low-substituted sodium hydroxymethyl cellulose, sodium carboxymethyl starch, and crospovidone;
in some embodiments, the diluent is selected from one or more of starch, pregelatinized starch, dextrin, lactose monohydrate, anhydrous lactose, sucrose, microcrystalline cellulose, inorganic salts, and sugar alcohols; preferably, the diluent is one or more of pregelatinized starch, dextrin, lactose, sucrose, microcrystalline cellulose, calcium sulphate, calcium hydrogenphosphate dihydrate, anhydrous calcium hydrogenphosphate, calcium phosphate, calcium carbonate, calcium stearate, magnesium oxide, aluminium hydroxide, mannitol, xylitol, and sorbitol; more preferably, the diluent is one or more of pregelatinized starch, lactose, sucrose, microcrystalline cellulose, calcium hydrogenphosphate dihydrate, anhydrous calcium hydrogenphosphate, calcium phosphate, mannitol, and dextrin;
in some embodiments, the lubricant is selected from one or more of talc, stearic acid, metal stearate, stearate, glyceryl behenate, sodium lauryl sulphate or colloidal silica; in some embodiments, the lubricant is selected from one or more of talc, stearic acid, metal stearate, stearate, glyceryl behenate, and sodium lauryl sulphate; preferably, the lubricant is one or more of talc, calcium stearate, magnesium stearate and zinc stearate, polyoxyethylene stearate, glyceryl monostearate, and glyceryl palmitostearate; more preferably, the lubricant is one or more of talc, calcium stearate, magnesium stearate, and glyceryl monostearate; more preferably, the lubricant is one or more of magnesium stearate, talc or colloidal silica;
in some embodiments, the weight ratio of the active ingredient to the infiltrating agent is 1:0.05 to 1:5, preferably 1:0.08 to 1:3, further preferably 1:0.12 to 1:2, and more preferably 1:0.125, 1:0.2, 1:0.25, 1:0.375, 1:0.5, 1:0.5, 1:1, 1:1.5, or 1:2;
in some embodiments, the weight ratio of the active ingredient to the diluent is 1:0.05 to 1:10, preferably 1:0.1 to 1:8.5, further preferably 1:0.18 to 1:8.5, and more preferably 1:0.18125, 1:0.1875, 1:0.9875, 1:1, 1:1.1125, 1:1.125, 1:1.2375, 1:1.25, 1:1.3625, 1:1.375, 1:3.725, 1:3.75, 1:6.45, 1:6.95, 1:7.45, 1:7.95, 1:8.25, or 1:8.45;
in some embodiments, the weight ratio of the active ingredient to the disintegrant is 1:0.01 to 1:3, preferably 1:0.03 to

1:1.5, further preferably 1:0.03 to 1:0.6, and more preferably 1:0.0375, 1:0.0625, 1:0.075, 1:0.125, 1:0.15, 1:0.25, or 1:0.5;

in some embodiments, the weight ratio of the active ingredient to the lubricant is 1:0.001 to 1:2, preferably 1:0.006 to 1:0.1, and more preferably 1:0.00625, 1:0.0125, 1:0.025, 1:0.05, or 1:0.1. The present invention provides a pharmaceutical composition, comprising an active ingredient and lactose, microcrystalline cellulose, low-substituted hydroxypropyl cellulose and magnesium stearate.

The present invention provides a pharmaceutical composition, comprising an active ingredient and silica, lactose, microcrystalline cellulose, low-substituted hydroxypropyl cellulose and magnesium stearate.

The present invention provides a pharmaceutical composition, comprising an active ingredient and silica, talc, lactose, microcrystalline cellulose, low-substituted hydroxypropyl cellulose and magnesium stearate.

The present invention provides a pharmaceutical composition, comprising an active ingredient and microcrystalline cellulose, mannitol, crospovidone and magnesium stearate.

The present invention provides a pharmaceutical composition, comprising an active ingredient and silica, magnesium silicate, microcrystalline cellulose, mannitol, crospovidone and magnesium stearate.

The present invention provides a pharmaceutical composition, comprising an active ingredient and pregelatinized starch, lactose, sodium carboxymethyl starch and talc.

The present invention provides a pharmaceutical composition, comprising an active ingredient and silica, pregelatinized starch, lactose, sodium carboxymethyl starch and talc.

The present invention provides a pharmaceutical composition, comprising an active ingredient and microcrystalline cellulose, lactose, croscarmellose sodium and magnesium stearate.

The present invention provides a pharmaceutical composition, comprising an active ingredient and silica, microcrystalline cellulose, lactose, croscarmellose sodium and magnesium stearate.

The present invention provides a pharmaceutical composition, comprising an active ingredient and lactose, microcrystalline cellulose, croscarmellose sodium and magnesium stearate.

The present invention provides a pharmaceutical composition, comprising an active ingredient and lactose, croscarmellose sodium and magnesium stearate.

The present invention provides a pharmaceutical composition, comprising an active ingredient and microcrystalline cellulose, croscarmellose sodium and magnesium stearate.

The present invention provides a pharmaceutical composition, comprising an active ingredient and mannitol, low-substituted carboxymethyl cellulose sodium and talc.

The present invention provides a pharmaceutical composition, comprising an active ingredient and microcrystalline cellulose, low-substituted carboxymethyl cellulose sodium and talc.

The present invention provides a pharmaceutical composition, comprising an active ingredient and mannitol, microcrystalline cellulose, low-substituted carboxymethyl cellulose sodium and talc.

The present invention provides a pharmaceutical composition, comprising an active ingredient and pregelatinized starch, sodium carboxymethyl starch and magnesium stearate.

The present invention provides a pharmaceutical composition, comprising an active ingredient and dextrin, sodium carboxymethyl starch and magnesium stearate.

The present invention provides a pharmaceutical composition, comprising an active ingredient and pregelatinized starch, dextrin, sodium carboxymethyl starch and magnesium stearate.

The present invention provides a pharmaceutical composition, comprising an active ingredient and calcium phosphate, crospovidone and talc.

The present invention provides a pharmaceutical composition, comprising an active ingredient and sucrose, crospovidone and talc.

The present invention provides a pharmaceutical composition, comprising an active ingredient and calcium phosphate, sucrose, crospovidone and talc. The present invention provides a pharmaceutical composition, comprising 10 to 80 w/w%, 10 to 60 w/w%, 10 to 45 w/w%, 10 to 40 w/w%, 5 to 35 w/w%, 5 to 20 w/w%, 10%, 20%, 40%, or 80% of an active ingredient.

The present invention provides a pharmaceutical composition, comprising 10 to 85 w/w%, 10 to 80 w/w%, 10 to 70 w/w%, 10 to 60 w/w%, 10 to 50 w/w%, 10 to 40 w/w%, 10 to 30 w/w%, 14.5%, 15%, 39.5%, 40%, 44.5%, 45%, 49.5%, 50%, 54.5%, 55%, 64.5%, 69.5%, 74.5%, 75%, 79.5%, 80%, 82.5%, or 84.5% of a diluent.

The present invention provides a pharmaceutical composition, comprising 1 to 8 w/w%, 1 to 5 w/w%, 1 to 3 w/w%, 3 to 5 w/w%, 5 to 8 w/w%, 3 w/w%, 5 w/w%, or 8 w/w% of a disintegrant.

The present invention provides a pharmaceutical composition, comprising 0.1 to 2 w/w%, 0.1 to 1.5 w/w%, 0.1 to 1 w/w%, 0.1 to 0.5 w/w%, 0.5-2 w/w%, 0.5 to 1 w/w%, 0.5 w/w%, 1 w/w%, or 2 w/w% of a lubricant.

The present invention provides a pharmaceutical composition, comprising 2 to 35 w/w%, 2 to 30 w/w%, 2 to 25 w/w%, 2 to 20 w/w%, 2 to 15 w/w%, 2 to 10 w/w%, 2 to 5 w/w%, 5 to 35 w/w%, 5 to 30 w/w%, 5 to 25 w/w%, 5 to 20 w/w%, 5 to

15 w/w%, 5 to 10 w/w%, 2 w/w%, 5 w/w%, 10 w/w%, 15 w/w%, or 20 w/w% of an infiltrating agent.

The pharmaceutical composition according to any one aspect of the present invention comprises the active ingredients and the non-active ingredients of any one of the preceding embodiments, including:

an active ingredient;
a diluent, wherein the diluent is selected from one or more of pregelatinized starch, lactose, microcrystalline cellulose, mannitol, dextrin, calcium phosphate or sucrose;
a disintegrant, wherein the disintegrant is selected from one or more of croscarmellose sodium, sodium carboxymethyl starch, crospovidone or low-substituted hydroxypropyl cellulose;
a lubricant, wherein the lubricant is selected from one or more of magnesium stearate, talc or colloidal silica;
optionally, the pharmaceutical composition further comprises an infiltrating agent, wherein the infiltrating agent is selected from one or more of silica, talc, and magnesium silicate.

The present invention provides a pharmaceutical composition, comprising 5 to 35 w/w% of an active ingredient, 60 to 85 w/w% of a diluent, 1 to 8 w/w% of a disintegrant, and 0.1 to 2 w/w% of a lubricant.

The present invention provides a pharmaceutical composition, comprising 5 to 20 w/w% of an active ingredient, 70 to 85 w/w% of a diluent, 1 to 8 w/w% of a disintegrant, and 0.1 to 2 w/w% of a lubricant.

The present invention provides a pharmaceutical composition, comprising 10 to 80 w/w% of an active ingredient, 2 to 35 w/w% of an infiltrating agent, 15 to 85 w/w% of a diluent, 1 to 10 w/w% of a disintegrant, and 0.1 to 5 w/w% of a lubricant.

The present invention provides a pharmaceutical composition, comprising 10 to 60 w/w% of an active ingredient, 2 to 35 w/w% of an infiltrating agent, 25 to 80 w/w% of a diluent, 1 to 10 w/w% of a disintegrant, and 0.1 to 5 w/w% of a lubricant.

The present invention provides a pharmaceutical composition, comprising 10 to 45 w/w% of an active ingredient, 2 to 25 w/w% of an infiltrating agent, 30 to 70 w/w% of a diluent, 1 to 10 w/w% of a disintegrant, and 0.1 to 5 w/w% of a lubricant.

The present invention provides a pharmaceutical composition, comprising 10 to 40 w/w% of an active ingredient, 5 to 20 w/w% of an infiltrating agent, 35 to 60 w/w% of a diluent, 1 to 10 w/w% of a disintegrant, and 0.1 to 5 w/w% of a lubricant. The present invention provides a pharmaceutical composition, comprising 10 w/w% of an active ingredient, 24.5 w/w% of lactose, 60 w/w% of microcrystalline cellulose, 5 w/w% of low-substituted hydroxypropyl cellulose, and 0.5 w/w% of magnesium stearate.

The present invention provides a pharmaceutical composition, comprising 10 w/w% of an active ingredient, 2 w/w% of silica, 24.5 w/w% of lactose, 58 w/w% of microcrystalline cellulose, 5 w/w% of low-substituted hydroxypropyl cellulose, and 0.5 w/w% of magnesium stearate.

The present invention provides a pharmaceutical composition, comprising 10 w/w% of an active ingredient, 5 w/w% of silica, 24.5 w/w% of lactose, 55 w/w% of microcrystalline cellulose, 5 w/w% of low-substituted hydroxypropyl cellulose, and 0.5 w/w% of magnesium stearate.

The present invention provides a pharmaceutical composition, comprising 10 w/w% of an active ingredient, 10 w/w% of silica, 24.5 w/w% of lactose, 50 w/w% of microcrystalline cellulose, 5 w/w% of low-substituted hydroxypropyl cellulose, and 0.5 w/w% of magnesium stearate.

The present invention provides a pharmaceutical composition, comprising 10 w/w% of an active ingredient, 10 w/w% of silica, 5 w/w% of talc, 24.5 w/w% of lactose, 45 w/w% of microcrystalline cellulose, 5 w/w% of low-substituted hydroxypropyl cellulose, and 0.5 w/w% of magnesium stearate.

The present invention provides a pharmaceutical composition, comprising 10 w/w% of an active ingredient, 10 w/w% of silica, 10 w/w% of talc, 24.5 w/w% of lactose, 40 w/w% of microcrystalline cellulose, 5 w/w% of low-substituted hydroxypropyl cellulose, and 0.5 w/w% of magnesium stearate.

The present invention provides a pharmaceutical composition, comprising 20 w/w% of an active ingredient, 29.5 w/w% of mannitol, 45 w/w% of microcrystalline cellulose, 5 w/w% of crospovidone, and 0.5 w/w% of magnesium stearate.

The present invention provides a pharmaceutical composition, comprising 40 w/w% of an active ingredient, 19.5 w/w% of mannitol, 35 w/w% of microcrystalline cellulose, 5 w/w% of crospovidone, and 0.5 w/w% of magnesium stearate.

The present invention provides a pharmaceutical composition, comprising 80 w/w% of an active ingredient, 4.5 w/w% of mannitol, 10 w/w% of microcrystalline cellulose, 5 w/w% of crospovidone, and 0.5 w/w% of magnesium stearate.

The present invention provides a pharmaceutical composition, comprising 40 w/w% of an active ingredient, 3 w/w% of silica, 2 w/w% of magnesium silicate, 19.5 w/w% of mannitol, 30 w/w% of microcrystalline cellulose, 5 w/w% of crospovidone, and 0.5 w/w% of magnesium stearate.

The present invention provides a pharmaceutical composition, comprising 40 w/w% of an active ingredient, 6 w/w% of silica, 4 w/w% of magnesium silicate, 19.5 w/w% of mannitol, 25 w/w% of microcrystalline cellulose, 5 w/w% of crospovidone, and 0.5 w/w% of magnesium stearate.

The present invention provides a pharmaceutical composition, comprising 40 w/w% of an active ingredient, 10 w/w% of silica, 5 w/w% of magnesium silicate, 19.5 w/w% of mannitol, 20 w/w% of microcrystalline cellulose, 5 w/w% of crospovidone, and 0.5 w/w% of magnesium stearate.

The present invention provides a pharmaceutical composition, comprising 20 w/w% of an active ingredient, 40 w/w% of pregelatinized starch, 35 w/w% of lactose, 3 w/w% of sodium carboxymethyl starch, and 2 w/w% of talc.

The present invention provides a pharmaceutical composition, comprising 40 w/w% of an active ingredient, 30 w/w% of pregelatinized starch, 25 w/w% of lactose, 3 w/w% of sodium carboxymethyl starch, and 2 w/w% of talc.

The present invention provides a pharmaceutical composition, comprising 80 w/w% of an active ingredient, 10 w/w% of pregelatinized starch, 5 w/w% of lactose, 3 w/w% of sodium carboxymethyl starch, and 2 w/w% of talc.

The present invention provides a pharmaceutical composition, comprising 40 w/w% of an active ingredient, 5 w/w% of silica, 30 w/w% of pregelatinized starch, 20 w/w% of lactose, 3 w/w% of sodium carboxymethyl starch, and 2 w/w% of talc.

The present invention provides a pharmaceutical composition, comprising 40 w/w% of an active ingredient, 10 w/w% of silica, 25 w/w% of pregelatinized starch, 20 w/w% of lactose, 3 w/w% of sodium carboxymethyl starch, and 2 w/w% of talc.

The present invention provides a pharmaceutical composition, comprising 40 w/w% of an active ingredient, 15 w/w% of silica, 20 w/w% of pregelatinized starch, 20 w/w% of lactose, 3 w/w% of sodium carboxymethyl starch, and 2 w/w% of talc.

The present invention provides a pharmaceutical composition, comprising 20 w/w% of an active ingredient, 45 w/w% of microcrystalline cellulose, 29.5 w/w% of lactose, 5 w/w% of croscarmellose sodium, and 0.5 w/w% of magnesium stearate.

The present invention provides a pharmaceutical composition, comprising 40 w/w% of an active ingredient, 35 w/w% of microcrystalline cellulose, 19.5 w/w% of lactose, 5 w/w% of croscarmellose sodium, and 0.5 w/w% of magnesium stearate.

The present invention provides a pharmaceutical composition, comprising 80 w/w% of an active ingredient, 10 w/w% of microcrystalline cellulose, 4.5 w/w% of lactose, 5 w/w% of croscarmellose sodium, and 0.5 w/w% of magnesium stearate.

The present invention provides a pharmaceutical composition, comprising 40 w/w% of an active ingredient, 5 w/w% of silica, 30 w/w% of microcrystalline cellulose, 19.5 w/w% of lactose, 5 w/w% of croscarmellose sodium, and 0.5 w/w% of magnesium stearate.

The present invention provides a pharmaceutical composition, comprising 40 w/w% of an active ingredient, 10 w/w% of silica, 25 w/w% of microcrystalline cellulose, 19.5 w/w% of lactose, 5 w/w% of croscarmellose sodium, and 0.5 w/w% of magnesium stearate.

The present invention provides a pharmaceutical composition, comprising 40 w/w% of an active ingredient, 15 w/w% of silica, 20 w/w% of microcrystalline cellulose, 19.5 w/w% of lactose, 5 w/w% of croscarmellose sodium, and 0.5 w/w% of magnesium stearate.

The present invention provides a pharmaceutical composition, comprising 10 w/w% of an active ingredient, 22 w/w% of lactose, 62 w/w% of microcrystalline cellulose, 5 w/w% of croscarmellose sodium, and 1 w/w% of magnesium stearate.

The present invention provides a pharmaceutical composition, comprising 25 w/w% of an active ingredient, 30 w/w% of mannitol, 40 w/w% of microcrystalline cellulose, 3 w/w% of low-substituted carboxymethyl cellulose sodium, and 2 w/w% of talc.

The present invention provides a pharmaceutical composition, comprising 25 w/w% of an active ingredient, 37.5 w/w% of pregelatinized starch, 35 w/w% of dextrin, 1.5 w/w% of sodium carboxymethyl starch, and 1 w/w% of magnesium stearate.

The present invention provides a pharmaceutical composition, comprising 33 w/w% of an active ingredient, 27 w/w% of calcium phosphate, 38.33 w/w% of sucrose, 1 w/w% of crospovidone, and 0.67 w/w% of talc. **In** any one of the above-mentioned pharmaceutical compositions, the active ingredient is selected from a compound of formula (I), or a stereoisomer or a pharmaceutically acceptable salt thereof.

**In** one aspect, the present invention provides a pharmaceutical formulation, comprising any one of the above-mentioned pharmaceutical compositions;

in some embodiments, the amount of the active ingredient in a unit pharmaceutical formulation is from 1 mg to 100 mg; in some embodiments, the amount of the active ingredient in a unit pharmaceutical formulation is 5 mg, 10 mg, 20 mg, 25 mg, 30 mg, 40 mg, 50 mg, 60 mg, 70 mg, 80 mg, 90 mg, or 100 mg.

**In** some embodiments, the formulation form of the pharmaceutical formulation is selected from a tablet, a granule, a capsule, a dry suspension, an oral solution, a soft capsule, and an emulsion; in some embodiments, the formulation form of the pharmaceutical formulation is selected from a tablet, a granule, a capsule, and a soft capsule.

In one aspect, the present invention provides the use of any one of the above-mentioned pharmaceutical

compositions or any one of the above-mentioned pharmaceutical formulations in the preparation of a medicament for treating an AAK1-related disease by inhibition or degradation of AAK1, wherein the disease is pain, preferably inflammatory pain, postoperative pain, trigeminal neuralgia, acute postherpetic neuralgia and postherpetic neuralgia, diabetic peripheral neuralgia, causalgia, occipital neuralgia, fibromyalgia, phantom limb pain, burn pain and other forms of neuralgia, neuropathy and spontaneous pain syndrome.

The preparation process of the pharmaceutical composition or the pharmaceutical formulation of the present invention is one or more of direct mixing, wet granulation, dry granulation, fluidized bed granulation, spray drying, freeze drying, hot melt extrusion, and pellet coating, preferably direct mixing, wet granulation, fluidized bed granulation, dry granulation and spray drying, and more preferably direct mixing and dry granulation.

In some embodiments, the preparation process is one or more of direct mixing, wet granulation, dry granulation, fluidized bed granulation, spray drying, freeze drying, hot melt extrusion, pellet coating, and extrusion spheronization, preferably spray drying and hot melt extrusion;

in some embodiments, the preparation process includes dry granulation for tableting, direct powder compression for tableting, and direct powder filling into capsules.

In some embodiments, the preparation process of the pharmaceutical composition further involves pretreatment of the active ingredient, wherein the pretreatment is selected from one or more of pulverization, solid dispersion, and nano-grinding.

Unless stated to the contrary, the terms used in both the description and the claims have the following meanings: The "pharmaceutically acceptable salt" refers to a salt that is safe, non-toxic, and neither biologically nor otherwise undesirable, is pharmaceutically acceptable for veterinary use and human pharmaceutical use, and has the desired pharmacological activity.

The term "stereoisomer" refers to an isomer produced as a result of different spatial arrangement of atoms in molecules, including cis-trans isomers, enantiomers and conformational isomers.

"Optional" or "optionally" or "alternative" or "alternatively" means that the events or conditions subsequently described may but not necessarily occur, and the description includes the case where the events or conditions occur and do not occur. For example, "heterocyclyl alternatively substituted with alkyl" means that the alkyl may but not necessarily exist, and the description includes the case where the heterocyclyl is substituted with alkyl and the case where the heterocyclyl is not substituted with alkyl.

## Brief Description of the Drawings

Figure 1 is a time-mechanical pain threshold (MPT) curve of the mice in Example 5.
Figure 2 is a dissolution curve of the tablet in Example 7.
Figure 3 is a dissolution curve of the capsule in Example 7.

## Detailed Description of Embodiments

The technical problems to be solved, technical solutions and beneficial effects of the present invention are described below in conjunction with examples. It should be understood that the specific examples described herein are merely used to explain the present invention, but are not intended to limit the present invention.

A compound of general formula (I) is prepared according to the following synthesis scheme:

### Example 1: Preparation of active ingredients

**Intermediate 1:**

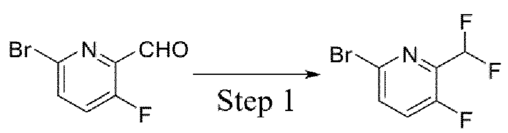


1A

**Intermediate 1**

Step 1:

Raw material 1A (10 g, 49 mmol) was dissolved in 200 mL of dichloromethane, and the mixture was cooled to -20°C. DAST (11.7 mL, 88 mmol) was added and the resulting mixture was slowly warmed to room temperature and reacted for 5 h. Upon complete depletion of raw materials monitored by TLC, the reaction was quenched with saturated aqueous sodium bicarbonate solution. The resulting reaction mixture was extracted with ethyl acetate and the organic phase was subjected to rotary evaporation. Then the residue was purified by silica gel column (petroleum ether : ethyl acetate = 20:1) to obtain the target compound **intermediate 1** (9.8 g, 89%).

$^1$H NMR (400 MHz, $CDCl_3$) δ7.65 - 7.58 (m, 1H), 7.46 - 7.40 (m, 1H), 6.85 - 6.56 (m, 1H).

**Intermediate 2:**

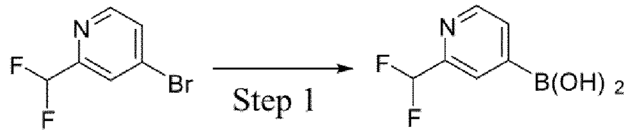


2A

**Intermediate 2**

Step 1:

2A (5 g, 24 mmol), Xphos PdG2 (189 mg, 0.24 mmol, CAS: 1310584-14-5), Xphos (229 mg, 0.48 mmol, CAS 564483-18-7), bis(pinacolato)diboron (9.14 g, 36 mmol) and KOAc (7.07 g, 72 mmol) were added to a flask. After nitrogen replacement, 200 mL of ethanol was added and the mixture was heated to 80°C and reacted for 5 h. Upon complete depletion of raw materials monitored by TLC, water was added to quench the reaction. The system was subjected to rotary evaporation to remove ethanol and then extracted with ethyl acetate. The organic phase was subjected to rotary evaporation to obtain **intermediate 2** (5.1 g).

LC-MS (ESI): m/z = 174.1 $[M+H]^+$.

**Intermediate 3:**

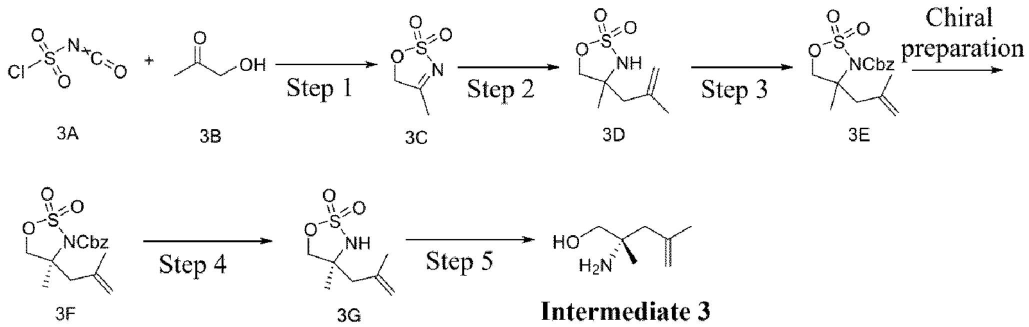


3A 3B 3C 3D 3E

3F 3G **Intermediate 3**

Step 1:

Under nitrogen atmosphere, chlorosulfonyl isocyanate (62 mL) was added to a three-necked round bottom flask, then 200 mL of dichloromethane was added, and the system was cooled to 0°C. 27 mL of formic acid was dissolved in 50 mL of dichloromethane. The mixture was slowly added to the system while the temperature was controlled at 0°C. After 30 minutes, the mixture was warmed to room temperature and stirred overnight. Hydroxyacetone (36.3 mL) and pyridine (58 mL) were dissolved in 1000 mL of dichloromethane. At 0°C, the mixture was slowly added dropwise to the system. After the addition was completed, the system was warmed to room temperature and stirred overnight. The system was subjected to rotary evaporation to remove the organic solvent and the residue was purified by silica gel column (eluent: dichloromethane) to obtain the title compound **3C** (36 g, 56%).

$^1$H NMR (400 MHz, $CDCl_3$) δ 5.06 (s, 2H), 2.42 (s, 3H).

Step 2:

Under nitrogen atmosphere, **3C** (36 g, 267 mmol) was dissolved in 800 mL of methyl tert-butyl ether. The system

was cooled to 0°C and then a solution of 2-methylallylmagnesium chloride in tetrahydrofuran (0.55 L, 0.5 M) was added dropwise. Upon complete depletion of raw materials monitored by TLC, the reaction was quenched by the addition of saturated aqueous ammonium chloride solution, and the resulting mixture was extracted with ethyl acetate and then subjected to rotary evaporation. The residue was purified by silica gel column to obtain the title compound **3D** (43 g, 84%).

$^1$H NMR (400 MHz, $CDCl_3$) δ 5.06 - 5.01 (m, 1H), 4.85 - 4.83 (m, 1H), 4.59 (s, 1H), 4.38 (d, 1H), 4.27 (d, 1H), 2.57 - 2.50 (m, 1H), 2.42 - 2.29 (m, 1H), 1.84 (s, 3H), 1.46 (s, 3H).

Step 3:

Under nitrogen, **3D** (1.91 g, 10 mmol) was dissolved in 50 mL of tetrahydrofuran. A solution of 1 M potassium tert-butoxide in tetrahydrofuran (15 mL) was added, followed by CbzCl (2.1 mL, 15 mmol). Upon complete depletion of raw materials monitored by TLC, the reaction was quenched by the addition of saturated aqueous ammonium chloride solution. The system was subjected to rotary evaporation to remove tetrahydrofuran, extracted with ethyl acetate, and then subjected to rotary evaporation. The residue was then purified by silica gel column (petroleum ether : ethyl acetate = 10:1) to obtain the title compound **3E** (2.6 g, 80%).

LC-MS (ESI): m/z = 343.0 $[M+NH_4]^+$.

120 g of **3E** was subjected to chiral preparation to obtain the target compound **3F** (55 g).

Preparation method: instrument: Waters SFC 150 Mgm, column: DAICEL CHIRALPAK OJ (250 mm × 50 mm, 10 μm); mobile phase: A for $CO_2$ and B for MeOH (BASE); gradient: 10% B; flow rate: 130 mL/min, back pressure: 100 bar; column temperature: 35°C; wavelength: 220 nm; cycle time: 4.5 min; sample preparation: sample concentration: 157.5 mg/ml, ethanol solution; sample injection: 0.8 ml/injection. After separation, the fractions were dried on a rotary evaporator at the bath temperature of 40°C to obtain compound **3F** (retention time: 0.680 minutes).

Step 4:

Compound **3F** (5 g, 15.4 mmol) was dissolved in 500 mL of methanol and 50 mg of 10% palladium on carbon catalyst was added. The mixture was subjected to hydrogen replacement. Upon the disappearing of fluorescence monitored by TLC, the system was filtered by suction to remove palladium on carbon from the system. The resulting filtrate was subjected to rotary evaporation to obtain the title compound **3G** (crude), which was directly used in the next step.

Step 5:

Compound **3G** was dissolved in 150 mL of tetrahydrofuran. At 0°C, lithium aluminium hydride (1.8 g, 47.4 mmol) was added in portions and the mixture was warmed to room temperature and stirred overnight. Water (1.8 mL), 10% aqueous sodium hydroxide solution (3.6 mL) and water (5.4 mL) were added and the mixture was stirred for 1 h. The resulting reaction mixture was filtered by suction to remove the solid. The resulting filtrate was subjected to rotary evaporation to obtain **intermediate 3** (crude product), which was directly used in the next reaction.

LC-MS (ESI): m/z = 130.1 $[M+H]^+$.

**Intermediate 4:**

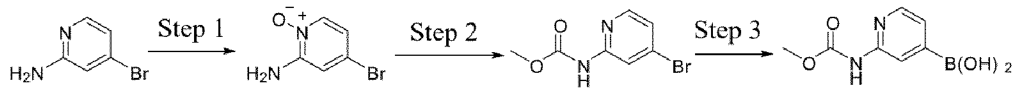


4A 4B 4C Intermediate 4

Step 1:

Compound **4A** (10 g, 57.8 mmol) was dissolved in 200 mL of acetone. At room temperature, m-chloroperoxybenzoic acid (11 g, 63.6 mmol) was dissolved in 200 mL of acetone and then the resulting mixture was added. The reaction mixture was stirred for 5 min to produce a large quantity of a solid. The solid was collected by suction filtration, washed with acetone, and dried to obtain compound **4B** (crude product, 10.7 g, 98%).

LC-MS (ESI): m/z = 189.0 and 191.0 $[M+H]^+$.

Step 2:

Compound **4B** (crude, 10.7 g) was dissolved in trimethyl orthoformate (200 mL) and 1.25 mL of boron trifluoride

diethyl etherate was added. The system was heated to 105°C and reacted overnight. The organic phase in the system was subjected to rotary evaporation, and then the residue was separated by column chromatography to obtain compound **4C** (9.1 g, 69%).

LC-MS (ESI): m/z = 231.0 and 233.0 $[M+H]^+$.

Step 3:

Compound **4C** (3.5 g, 15.1 mmol), Xphos PdG2 (600 mg, 0.76 mmol, CAS: 1310584-14-5), Xphos (700 mg, 1.47 mmol, CAS 564483-18-7), potassium acetate (4.5 g, 45.8 mmol) and bis(pinacolato)diboron (6 g, 23.6 mmol) were dissolved in 250 mL of ethanol in a round bottom flask. The system was subjected to nitrogen replacement, warmed to 80°C and reacted overnight. The system was subjected to rotary evaporation to remove ethanol and then extracted with ethyl acetate to obtain the title compound **intermediate 4** (4 g).

LC-MS (ESI): m/z = 197.1 $[M+H]^+$.

**Intermediate 5:**

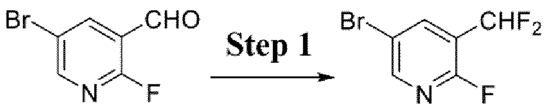


5A

Intermediate 5

Step 1:

Raw material **5A** (5.00 g, 24.51 mmol) was dissolved in 100 mL of dichloromethane, and the mixture was cooled to -20°C. DAST (6.5 mL, 49.02 mmol) was added, and the resulting mixture was slowly warmed to room temperature and reacted for 2 h. Upon complete depletion of raw materials monitored by TLC, the reaction was quenched with saturated aqueous sodium bicarbonate solution and extracted with dichloromethane. The organic phase was subjected to rotary evaporation and the residue was purified by silica gel column (petroleum ether : ethyl acetate = 20:1) to obtain **intermediate 5** (5.00 g, 90.27%).

$^1H$ NMR (400 MHz, $CDCl_3$) $\delta$ 8.40 (dd, 1H), 8.15 (dt, 1H), 6.95 - 6.67 (m, 1H).

**1.1 Preparation of compound 1**

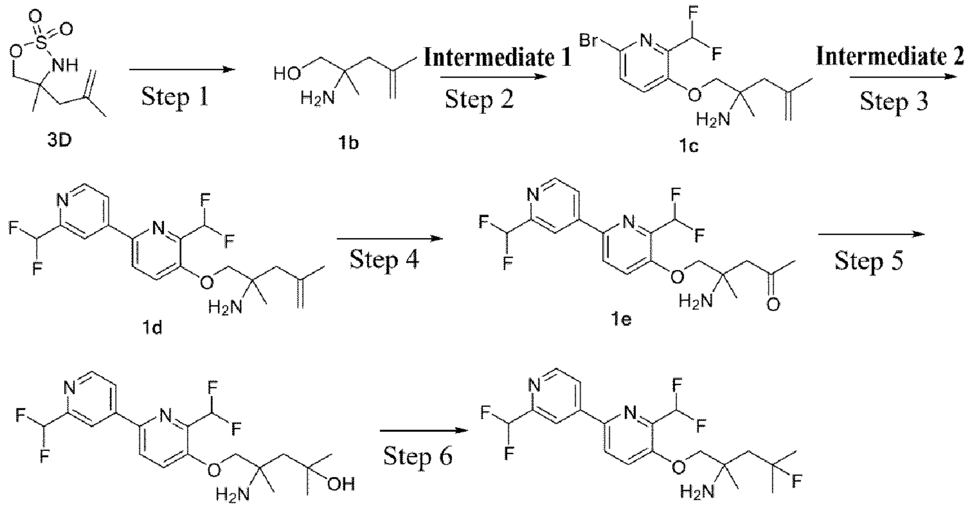


3D 1b 1c 1d 1e 1f Compound 1

Step 1:

**3D** (8 g, 42 mmol) was dissolved in 500 mL of tetrahydrofuran. The system was cooled to 0°C and lithium aluminium hydride (3.99 g, 105 mmol) was slowly added. Then the mixture was warmed to room temperature and reacted for 6 h. Water (4 mL), 8 M aqueous NaOH solution, and water (12 mL) were sequentially added and the mixture was stirred for 1 h. The resulting mixture was filtered by suction to remove the solid and the resulting filtrate was subjected to rotary evaporation to obtain the target compound **1b** (crude product, 9 g), which was directly used in the next step without purification.

LC-MS (ESI): m/z = 130.2 $[M+H]^+$.

Step 2:

Crude product **1b** (2 g) was added to a solution of potassium tert-butoxide in tetrahydrofuran (27 mL). At room temperature, the mixture was stirred for 5 min and then **intermediate 1** (4 g, 18 mmol) was added. The resulting mixture was subjected to nitrogen replacement, then heated to 80°C and reacted overnight. The organic phase in the system was subjected to rotary evaporation, and the residue was separated and purified by silica gel column chromatography (dichloromethane : methanol = 10:1) to obtain the target compound **1c** (1.1 g, 35%).

LC-MS (ESI): m/z = 335.1 and 337.1 $[M+H]^+$.

Step 3:

**Intermediate 2** (1.1 g, 3.3 mmol), 1c (880 mg, 5 mmol), potassium phosphate (9.2 g, 43 mmol), Xphos PdG2 (500 mg, 0.63 mmol, CAS: 1310584-14-5), and Xphos (650 mg, 1.36 mmol, CAS 564483-18-7) were added to a sealed tube, and 30 mL of tetrahydrofuran was added. After nitrogen replacement, the mixture was warmed to 80°C and reacted for 5 h. Upon complete depletion of raw materials monitored by TLC, the system was filtered by suction to remove the solid, which was then washed with methanol. The filtrate was collected and subjected to rotary evaporation, and the residue was separated and purified by silica gel column chromatography (dichloromethane : methanol = 10:1) to obtain the title compound **1d** (360 mg, 29%).

LC-MS (ESI): m/z = 384.2 $[M+H]^+$.

$^1$H NMR (400 MHz, DMSO-$d_6$) δ 8.80 - 8.76 (m, 1H), 8.42 - 8.36 (m, 1H), 8.32 (s, 1H), 8.24 - 8.18 (m, 1H), 7.84 - 7.79 (m, 1H), 7.42 - 6.88 (m, 2H), 4.87 (s, 1H), 4.72 (s, 1H), 3.88 (s, 2H), 2.22 (s, 2H), 1.78 (s, 3H), 1.15 (s, 3H).

Step 4:

**1d** (360 mg, 0.94 mmol) was dissolved in 20 mL of dichloromethane. The mixture was cooled to -60°C and ozone was introduced. Upon complete depletion of raw materials monitored by TLC, 1 g of triphenylphosphine was added and the system was warmed to room temperature and stirred for 15 min. The organic phase was subjected to rotary evaporation and the residue was separated and purified by silica gel column chromatography (dichloromethane : methanol = 10:1) to obtain the title compound **1e** (300 mg, 83%).

LC-MS (ESI): m/z = 386.2 $[M+H]^+$.

Step 5:

Under nitrogen atmosphere, **1e** (300 mg, 0.78 mmol) was dissolved in 20 mL of tetrahydrofuran and the system was cooled to 0°C. A solution of methylmagnesium bromide in THF (1 mL, 3 M) was added and the mixture was slowly warmed to room temperature. Upon complete depletion of raw materials monitored by TLC, the reaction was quenched by the addition of saturated aqueous ammonium chloride solution, and extracted with dichloromethane. The organic phase was subjected to rotary evaporation to obtain the title compound **1f** (240 mg, 0.6 mmol), which was directly used in the next step.

LC-MS (ESI): m/z = 402.2 $[M+H]^+$.

Step 6:

Under nitrogen atmosphere, **1f** (240 mg, 0.6 mmol) was dissolved in 15 mL of dichloromethane and the mixture was cooled to -78°C. DAST (0.4 mL, 2.8 mmol) was added and the system was slowly warmed to room temperature. Upon complete depletion of raw materials monitored by TLC, the reaction was quenched by the addition of saturated aqueous sodium bicarbonate solution and extracted with dichloromethane. The organic phase was subjected to rotary evaporation and then the resulting product was separated by HPLC and lyophilized to obtain the title compound **1** (110 mg, 42%).

LC-MS (ESI): m/z = 404.2 $[M+H]^+$.
$^1$H NMR (400 MHz, DMSO-$d_6$) δ 8.82 - 8.76 (m, 1H), 8.42 - 8.36 (m, 1H), 8.32 (s, 1H), 8.23 - 8.18 (m, 1H), 7.81 - 7.75 (m, 1H), 7.41 - 6.89 (m, 2H), 3.95 (s, 2H), 1.94 - 1.86 (m, 2H), 1.49 - 1.36 (m, 6H), 1.23 (s, 3H).

**1.2 Preparation of compounds 2 and 3**

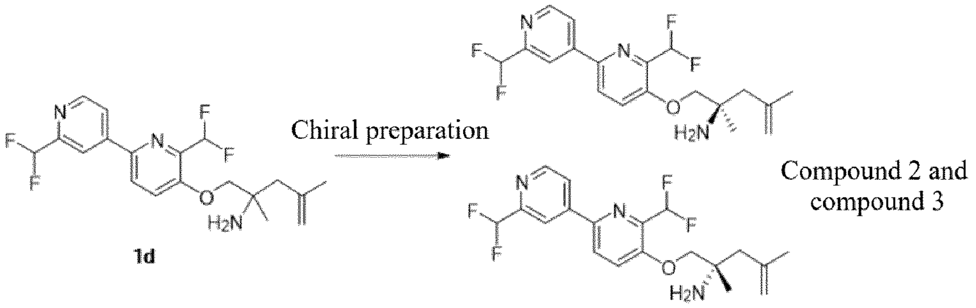

1d
Compound 2 and compound 3

**1d** (80 mg) was subjected to chiral resolution to obtain compound **2** (33.7 mg) and compound **3** (25.3 mg).

Preparation method:

instrument: SHIMADZU LC-20AP, column: DAICEL CHIRALPAK IG (250 mm × 30 mm, 10 μm); mobile phase: A: n-hexane, B: ethanol (0.1% $NH_3$•$H_2O$);; gradient: 8% B gradient elution; flow rate: 120 mL/min, column temperature: 25°C, wavelength: 254 nm, cycle time: 16 min; sample preparation: sample concentration: 1.5 mg/ml, ethanol solution; sample injection: 2 ml/injection. After separation, the fractions were dried on a rotary evaporator at the bath temperature of 40°C to obtain P1 (retention time: 2.658 minutes, set to be compound 2) and P2 (retention time: 4.205 minutes, set to be compound 3).

**1.3 Preparation of compound 4**

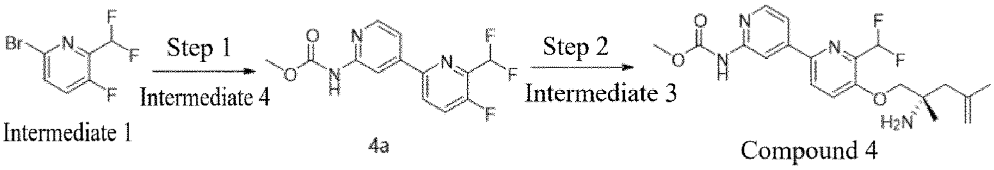

Intermediate 1
4a
Compound 4

Step 1:

**Intermediate 4** (500 mg, 1.8 mmol), **intermediate 1** (500 mg, 2.2 mmol), Xphos PdG2 (200 mg, 0.25 mmol, CAS: 1310584-14-5), Xphos (250 mg, 0.52 mmol, CAS 564483-18-7), and potassium phosphate (4.5 g, 21.2 mmol) were added to a sealed tube, and 20 mL of tetrahydrofuran was added. After nitrogen replacement, the system was warmed to 80°C and reacted for 3 h. The resulting reaction mixture was mixed with silica gel and separated by column chromatography to obtain compound **4a** (197 mg, 37%).
LC-MS (ESI): m/z = 298.1 $[M+H]^+$.

Step 2:

Compound **4a** (197 mg, 0.66 mmol), **intermediate 3** (90 mg, 0.7 mmol) and 1 mL of potassium tert-butoxide (1 M in THF) were added to a sealed tube. After nitrogen replacement, the system was warmed to 80°C and reacted for 3 h. The reaction solution was concentrated to dryness, and the residue was separated and purified by preparative chromatography to obtain the target **compound 4** (30 mg, 11%).
LC-MS (ESI): m/z = 407.1 $[M+H]^+$.
$^1$H NMR (400 MHz, DMSO-$d_6$) δ 10.23 (s, 1H), 8.49 (s, 1H), 8.38 - 8.33 (m, 1H), 8.18 - 8.13 (m, 1H), 7.79 - 7.75 (m,

1H), 7.69 - 7.63 (m, 1H), 7.40 - 7.08 (m, 1H), 4.86 (s, 1H), 4.71 (s, 1H), 3.88 (s, 2H), 3.71 (s, 3H), 2.23 (s, 2H), 1.78 (s, 3H), 1.14 (s, 3H).

### 1.4 Preparation of compound 5

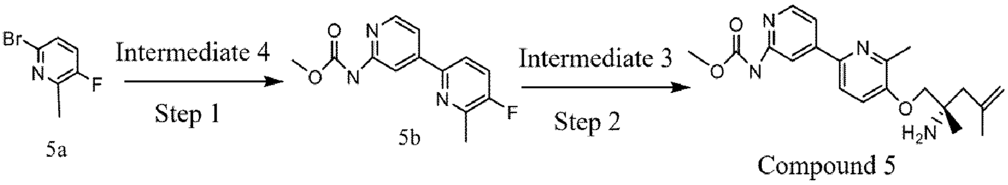


5a 5b Compound 5

Step 1:

**5a** (1.5 g, 7.89 mmol), **intermediate 4** (2.3 g, 11.84 mmol), potassium phosphate (21.8 g, 102.57 mmol), Xphos PdG2 (1.24 g, 1.58 mmol, CAS: 1310584-14-5), and Xphos (1.5 g, 3.16 mmol, CAS 564483-18-7) were added to a sealed tube, and 60 mL of tetrahydrofuran was added. After nitrogen replacement, the mixture was warmed to 80°C and reacted for 5 h. Upon complete depletion of raw materials monitored by TLC, the system was filtered by suction to remove the solid, which was then washed with methanol. The filtrate was collected and subjected to rotary evaporation, and the residue was purified by silica gel column (dichloromethane : methanol = 10:1) to obtain the title compound **5b** (1.4 g, 68%).

LC-MS (ESI): m/z = 262.0 $[M+H]^+$.

Step 2:

**Intermediate 3** (495 mg, 3.83 mmol) was added to 15 mL of DMF solution. Under an ice bath, NaH (275 mg, 11.49 mmol) was added and the mixture was stirred for 10 min. Then compound **5b** (1 g, 3.83 mmol) was added. After nitrogen replacement, the mixture was reacted at 0°C for 1 h. The reaction was quenched by the addition of water and the mixture was then extracted with ethyl acetate. The organic phase was subjected to rotary evaporation and the residue was purified by column chromatography (dichloromethane : methanol = 10:1) to obtain **compound** 5 (110 mg).

LC-MS (ESI): m/z = 371.2 $[M+H]^+$.

$^1$H NMR (400 MHz, DMSO-$d_6$) δ 10.16 (s, 1H), 8.47 (s, 1H), 8.29 (d, 1H), 7.80 (d, 1H), 7.62 (dd, 1H), 7.40 (d, 1H), 4.86 (s, 1H), 4.70 (s, 1H), 3.75 (s, 2H), 3.70 (s, 3H), 2.50 (s, 3H), 2.23 (s, 2H), 1.78 (s, 3H), 1.58 (s, 2H), 1.14 (s, 3H).

### 1.5 Preparation of compound 6

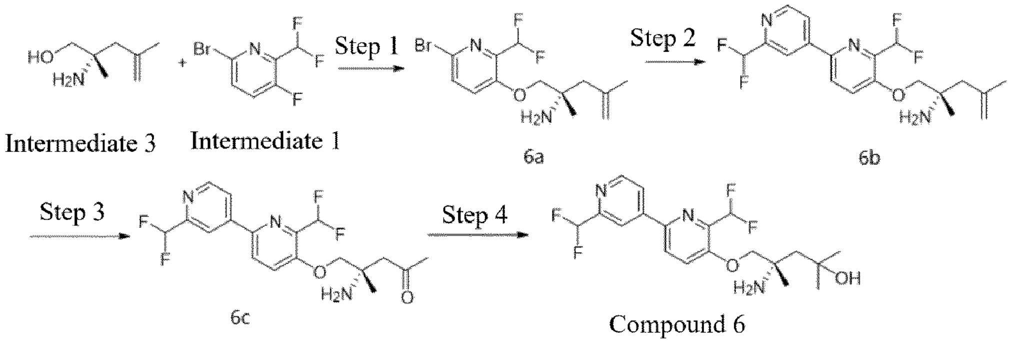


Intermediate 3 Intermediate 1 6a 6b 6c Compound 6

Step 1:

**Intermediate 3** (1 g, 7.7 mmol), **intermediate 1** (1.6 g, 7.1 mmol) and 12 mL of potassium tert-butoxide (1 M in THF) were added to a sealed tube. After nitrogen replacement, the system was warmed to 80°C and reacted for 3 h. The system was cooled to room temperature, mixed with silica gel, and separated by column chromatography (petroleum ether : ethyl acetate = 1:1 to ethyl acetate) to obtain the target compound **6a** (500 mg, 21%).

LC-MS (ESI): m/z = 355.1 $[M+H]^+$.

Step 2:

Compound **6a** (500 mg, 1.5 mmol), **intermediate 2** (620 mg, 3.6 mmol), Xphos PdG2 (200 mg, 0.25 mmol, CAS: 1310584-14-5), Xphos (250 mg, 0.52 mmol, CAS 564483-18-7), and potassium phosphate (4.5 g, 21.2 mmol) were added to a sealed tube, and 20 mL of tetrahydrofuran was added. After nitrogen replacement, the system was warmed to 80°C and reacted for 3 h. The resulting reaction mixture was mixed with silica gel and separated by column chromatography (petroleum ether : ethyl acetate = 1:1 to ethyl acetate) to obtain compound **6b** (350 mg, 61%).
LC-MS (ESI): m/z = 384.2 $[M+H]^+$.

Step 3:

Compound **6b** (350 mg, 0.91 mmol) was dissolved in 20 mL of dichloromethane. The system was cooled to -78°C and ozone was introduced. Upon complete depletion of raw materials monitored by TLC, excess triphenylphosphine was added and the mixture was slowly warmed to room temperature, mixed with silica gel, and separated by column chromatography (petroleum ether : ethyl acetate = 1:1 to ethyl acetate) to obtain compound **6c** (310 mg, 89%).
LC-MS (ESI): m/z = 386.1 $[M+H]^+$.

Step 4:

Compound 6c (160 mg, 0.42 mmol) was dissolved in 10 mL of tetrahydrofuran and the mixture was subjected to nitrogen replacement. At 0°C, 1.4 mL of methylmagnesium chloride (3 M, dissolved in THF) was added. The mixture was slowly warmed to room temperature, and the reaction was quenched by the addition of saturated aqueous ammonium chloride solution. The organic phase in the system was subjected to rotary evaporation, extracted with dichloromethane and then subjected to rotary evaporation. The residue was purified by preparative chromatography and then lyophilized to obtain compound 6 (30 mg, 18%).
LC-MS (ESI): m/z = 402.2 $[M+H]^+$.
$^1H$ NMR (400 MHz, DMSO-$d_6$) δ 8.81 - 8.78 (m, 1H), 8.42 - 8.37 (m, 1H), 8.32 (s, 1H), 8.23 - 8.19 (m, 1H), 7.81 - 7.74 (m, 1H), 7.41 - 6.88 (m, 2H), 4.02 - 3.91 (m, 2H), 1.71 (d, 1H), 1.60 (d, 1H), 1.27 (s, 3H), 1.23 (s, 3H), 1.16 (s, 3H).

**1.6 Preparation of compound 7**

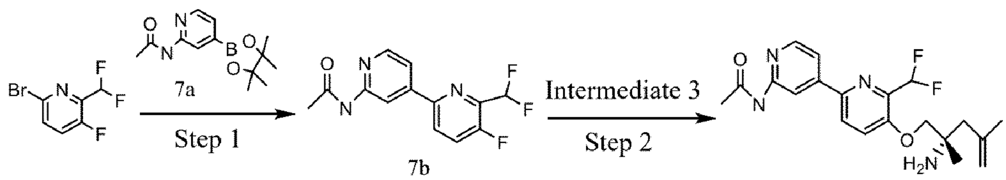


7a

7b

Compound 7

Step 1:

**7a** (900 mg), **intermediate 1** (633 mg, 2.8 mmol), Xphos PdG2 (250 mg, 0.32 mmol), Xphos (500 mg, 1.05 mmol), and potassium phosphate (6.0 g, 28.3 mmol) were added to a sealed tube, and 30 mL of tetrahydrofuran was added. After nitrogen replacement, the system was warmed to 80°C and reacted for 3 h. The resulting reaction mixture was mixed with silica gel and separated by column chromatography to obtain compound **7b** (428 mg, 54%).
LC-MS (ESI): m/z = 282.2 $[M+H]^+$.

Step 2:

Compound **7b** (200 mg, 0.71 mmol), **intermediate 3** (100 mg, 0.77 mmol) and 2.5 mL of potassium tert-butoxide (1 M in THF) were added to a sealed tube. After nitrogen replacement, the system was warmed to 80°C and reacted for 3 h. The reaction mixture was separated and purified by preparative chromatography to obtain compound 7 (89 mg, 32%).
LC-MS (ESI): m/z = 391.1 $[M+H]^+$.
$^1H$ NMR (400 MHz, DMSO-$d_6$) δ 10.54 (s, 1H ), 8.68 (s, 1H), 8.41 - 8.37 (m, 1H), 8.16 - 8.10 (m, 1H), 7.81 - 7.75 (m,

1H), 7.72 - 7.67 (m, 1H), 7.38 - 7.07(m, 1H), 4.86 (s, 1H), 4.71 (s, 1H), 3.88 (s, 2H), 2.23 (s, 2H), 2.12 (s, 3H), 1.78 (s, 3H), 1.14 (s, 3H).

**1.7 Preparation of compound 8**

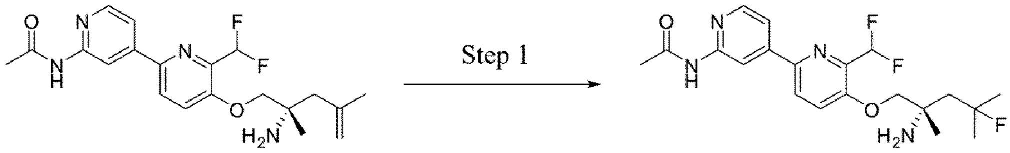


Compound 7 Compound 8

Step 1:

Ferric nitrate nonahydrate (133 mg, 0.33 mmol) was dissolved in water (3 mL) and the mixture was subjected to nitrogen replacement and then cooled to 0°C. Selective fluorination reagent (117 mg, 0.33 mmol) and 3 mL of acetonitrile were added. Compound **7** (35 mg, 0.09 mmol) was dissolved in 3 mL of acetonitrile and the resulting mixture was added to the system. The mixture was stirred for 5 min and then sodium borohydride (40 mg, 1.05 mmol) was added in portions. The resulting mixture was reacted for 30 min while the temperature was maintained at 0°C. Ammonia water (1 mL) was added to quench the reaction, followed by extraction with the mixed solvents of dichloromethane and methanol (10:1). After rotary evaporation, the residue was purified by preparative HPLC to obtain compound **8** (10 mg, 28%).

LC-MS (ESI): m/z = 411.3 $[M+H]^+$.

$^1H$ NMR (400 MHz, DMSO-$d_6$) δ 10.55 (s, 1H), 8.69 (s, 1H), 8.43 - 8.35 (m, 1H), 8.18 - 8.10 (m, 1H), 7.78 - 7.73 (m, 1H), 7.72 - 7.65 (m, 1H), 7.40 - 7.05 (m, 1H), 3.94 (s, 2H), 2.12 (s, 3H), 1.95 - 1.86 (m, 2H), 1.50 - 1.36 (m, 6H), 1.23 (s, 3H).

**1.8 Preparation of compound 9**

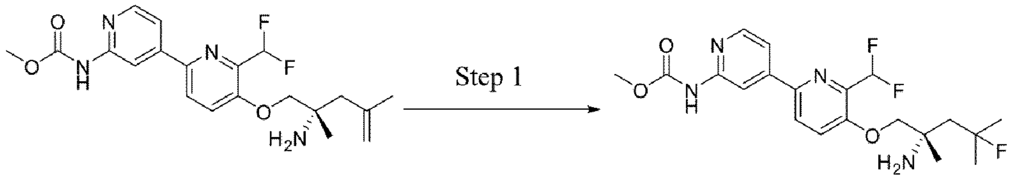


Compound 4 Compound 9

Step 1:

Ferric nitrate nonahydrate (324 mg, 0.8 mmol) was dissolved in water (7 mL) and the mixture was subjected to nitrogen replacement and then cooled to 0°C. Selective fluorination reagent (284 mg, 0.8 mmol) and 7 mL of acetonitrile were added and **compound 4** (81 mg, 0.2 mmol) was dissolved in 7 mL of acetonitrile. The resulting mixture was added to the system. The resulting mixture was stirred for 5 min and then sodium borohydride (100 mg, 2.6 mmol) was added in portions. The reaction mixture was reacted for 30 min while the temperature was maintained at 0°C. Ammonia water (2.5 mL) was added to quench the reaction, followed by extraction with the mixed solvents of dichloromethane: methanol (10:1). After rotary evaporation, the residue was purified by preparative HPLC to obtain compound **9** (9 mg, 11%).

LC-MS (ESI): m/z = 427.2 $[M+H]^+$.

$^1H$ NMR (400 MHz, DMSO-$d_6$) δ 10.25 (s, 1H), 8.49 (s, 1H), 8.38 - 8.33 (m, 1H), 8.19 - 8.13 (m, 1H), 7.78 - 7.72 (m, 1H), 7.68 - 7.64 (m, 1H), 7.38 - 7.09 (m, 1H), 3.94 (s, 2H), 3.71 (s, 3H), 1.95 - 1.86 (m, 2H), 1.50 - 1.37 (m, 6H), 1.23 (s, 3H).

**1.9 Preparation of compound 10**

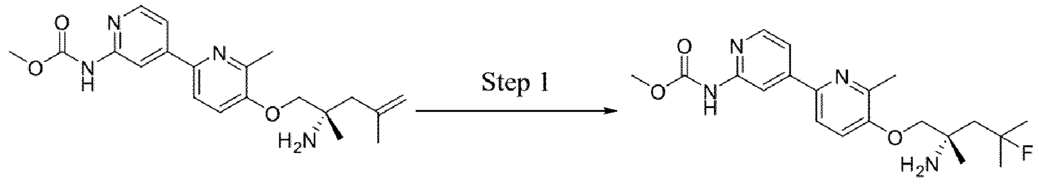


Compound 5 Compound 10

Ferric nitrate nonahydrate (88 mg, 0.22 mmol) was dissolved in water (2 mL). The mixture was ultra-sonicated for 5 min and cooled to 0°C. Then a solution of selective fluorination reagent (76 mg, 0.22 mmol) in 2 mL of acetonitrile was added, followed by a solution of **compound 5** (20 mg, 0.05 mmol) in 2 mL of acetonitrile. Sodium borohydride (30 mg, 0.79 mmol) was then added in portions. The mixture was reacted for 1 h. When the reaction of the raw materials was completed as shown by LC-MS, the reaction mixture was diluted by the addition of water and then extracted with dichloromethane. The organic phases were combined, dried and concentrated to obtain a crude, which was separated and purified by preparative chromatography to obtain **compound 10** (10 mg, 47%).

LC-MS (ESI): m/z = 391.3$[M+H]^+$.

$^1H$ NMR (400 MHz, $CD_3OD$) $\delta$ 8.42 (s, 1H), 8.27 (d, 1H), 7.76 (d, 1H), 7.61 (d, 1H), 7.40 (d, 1H), 3.98 (s, 2H), 3.80 (s, 3H), 2.57 (s, 3H), 2.09 (s, 1H), 2.04 (s, 1H), 1.50 (d, 3H), 1.45 (d, 3H), 1.40 (s, 3H).

**Example 2: *In vitro* AAK1 enzyme activity assay**

Compound stock solution (concentration: 10 mM, dissolved in DMSO) was diluted with DMSO to 0.2 mM and then diluted with DMSO in 5-fold gradient to obtain compound solutions with 10 concentrations. Subsequently, the compound solutions with different concentrations were diluted 50-fold in 1×kinase reaction buffer (containing 40 mM Tris, 20 mM $MgCl_2$, 0.1% BSA and 0.5 mM DTT) for later use. AAK1 (Signalchem, Cat# A01-11G-10) was diluted with 1×kinase reaction buffer to 2-fold the final concentration (final concentrations: 30 nM and 28 nM). AAK1 was added to a 384-well white plate at 2 μL/well, and the compounds were then added at 1 μL/well. The plate was sealed with a plate-sealing film, centrifuged at 1000 rpm for 30 seconds and then placed at room temperature for 10 minutes. A mixed solution of ATP (Promega, Cat# V914B) and substrate Micro2 (GenScript, Cat# PE0890) was formulated at 4-fold the final concentration (for AAK1, the corresponding final concentrations of ATP: 15 μM and 5 μM, and the corresponding final concentration of Micro2: 0.1 mg/mL). To the reaction plate was added the mixed solution of ATP and the substrate at 1 μL/well. The plate was sealed with a plate-sealing film and centrifuged at 1000 rpm for 30 seconds. The reaction was carried out at room temperature for 60 minutes (AAK1). ADP-Glo (Promega, Cat# V9102) was transferred to the 384-well plate at 4 μL/well and centrifugation was carried out at 1000 rpm for 1 minute. The mixture was incubated at 25°C for 40 minutes. A detection solution was transferred to the 384-well plate at 8 μL/well and centrifugation was carried out at 1000 rpm for 1 minute. The resulting mixture was incubated at 25°C for 40 minutes. The RLU (Relative luminescence unit) signal values were read using a Biotek multimode microplate reader and the percent inhibition was calculated according to the following formula: $[1-(LUM_{compound}-LUM_{positive\ control})/(LUM_{negative\ control}-LUM_{positive\ control})] \times 100$. $IC_{50}$ values were calculated using Graphpad 7.0 software using a four-parameter non-linear fit equation. The specific results are shown in Table 1.

Table 1 Inhibitory activity against AAK1

| Compound No. | $IC_{50}$/nM |
|---|---|
| Compound 1 | 14.72 |
| Compound 2 | 10.92 |
| Compound 5 | 11.47 |
| Compound 7 | 19.82 |
| Compound 8 | 22.26 |
| Compound 9 | 37.73 |
| Compound 10 | 13.74 |

Conclusion: the compounds of the present invention show relatively high inhibitory activity against the AAK1 receptor.

**Example 3: Pharmacokinetic test of dogs**

Experimental animals: male beagle dogs, about 8 to 11 kg, 6 beagle dogs/compound, purchased from Beijing Marshall Biotechnology Co., Ltd.

Experimental method: on the day of the experiment, 12 beagle dogs were randomly grouped according to their body weights. The animals were fasted with water available for 12 to 14 h one day before the administration and were fed 4 h after the administration. Administration was performed as per Table 2.

Table 2. Administration information

| Group | Number | Administration information | | | | | |
|---|---|---|---|---|---|---|---|
| | Male | Test compound | Administration dosage (mg/kg) | Administration concentration (mg/mL) | Administration volume (mL/kg) | Collected sample | Mode of administration |
| G1 | 3 | LX-9211 | 1 | 1 | 1 | Plasma | Intravenous administration |
| G2 | 3 | | 3 | 0.6 | 5 | Plasma | Intragastric administration |
| G3 | 3 | Compound 9 | 1 | 1 | 1 | Plasma | Intravenous administration |
| G4 | 3 | | 3 | 0.6 | 5 | Plasma | Intragastric administration |
| Note: vehicle for intravenous administration: 5% DMA + 5% Solutol + 90% Saline; vehicle for intragastric administration: 0.5% MC (DMA: dimethylacetamide; Solutol: polyethylene glycol-15-hydroxystearate; Saline: physiological saline; MC: methylcellulose) | | | | | | | |

Before and after the administration, 1 ml of blood was taken from the jugular veins or limb veins, and placed in an EDTAK2 centrifuge tube. Centrifugation was performed at 5000 rpm at 4°C for 10 min, and plasma was collected. Blood sampling time points for both the LX9211 intravenous administration group and intragastric administration group include: 0 min, 5 min, 15 min, 30 min, 1 h, 2 h, 4 h, 6 h, 8 h, 10 h, 12 h, and 24 h, and blood sampling time points for both the compound 9 intravenous administration group and intragastric administration group include: 0 min, 5 min, 15 min, 30 min, 1 h, 2 h, 4 h, 6 h, 8 h, 10 h, 12 h, 24 h, and 48 h. Before analysis and detection, all samples were stored at -80°C, and the samples were quantitatively analysed by LC-MS/MS. The experimental results are shown in Table 3.

Table 3. Pharmacokinetic parameters of test compounds in plasma of beagle dogs

| Test compound | Mode of administration | CL (mL/min/kg) | $Vd_{ss}$ (L/kg) | $AUC_{0-t}$ (hr×ng/mL) | F (%) |
|---|---|---|---|---|---|
| LX-9211 | i.v. (1 mg/kg) | 22.2 ± 7.7 | 9.08 ± 1.4 | 751 ± 204 | - |
| | i.g. (3 mg/kg) | - | - | 500 ± 183 | 22.2 ± 8.1 |
| Compound 9 | i.v. (1 mg/kg) | 39.8 ± 10 | 23.6 ± 3.8 | 398 ± 94 | - |
| | i.g. (3 mg/kg) | | | 1353 ± 83 | 113 ± 6.9 |
| -: not applicable. | | | | | |

Notes: LX-9211 has a structure of

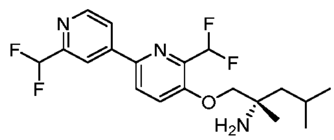

.

**Conclusion:** the compounds of the present invention possess good pharmacokinetic characteristics.

**Example 4: Test for hERG potassium ion channel**

Experimental platform: electrophysiological manual patch-clamp system

Cell line: Chinese hamster ovary (CHO) cell line stably expressing hERG potassium ion channel

Experimental method: in CHO (Chinese Hamster Ovary) cells stably expressing hERG potassium channel, the whole cell patch-clamp technique was used to record hERG potassium channel current at room temperature. The glass microelectrode was made of a glass electrode blank (BF150-86-10, Sutter) by a puller. The tip resistance after filling the liquid in the electrode was about 2-5 MΩ. The glass microelectrode can be connected to the patch-clamp amplifier by inserting the glass microelectrode into an amplifier probe. The clamping voltage and data recording were controlled and recorded by the pClamp 10 software through a computer. The sampling frequency was 10 kHz, and the filtering frequency was 2 kHz. After the whole cell records were obtained, the cells were clamped at -80 mV, and the step voltage that induced the hERG potassium current (I hERG) was depolarized from -80 mV to +20 mV for 2 s, then repolarized to -50 mV, and returned to -80 mV after 1 s. This voltage stimulation was given every 10 s, and the administration process was started after the hERG potassium current was confirmed to be stable (at least 1 minute). The compound was administered for at least 1 minute at each test concentration, and at least 2 cells ($n \geq 2$) were tested at each concentration.

Data processing: data analysis processing was carried out by using pClamp 10, GraphPad Prism 5 and Excel software. The inhibition degree of hERG potassium current (peak value of hERG tail current induced at -50 mV) at different compound concentrations was calculated by the following formula:

$$\text{Inhibition } \% = [1 - (I / Io)] \times 100\%$$

where Inhibition % represents the percentage of inhibition of hERG potassium current by the compound, and I and Io represent the amplitude of hERG potassium current after and before the administration, respectively.

Compound IC50 was calculated using GraphPad Prism 5 software by fitting according to the following equation:

$$Y = \text{Bottom} + (\text{Top-Bottom})/(1 + 10\text{^}((\text{LogIC50-X}) \times \text{HillSlope}))$$

Among the equation, X represents the Log value of the tested concentration of the test sample, Y represents the inhibition percentage at the corresponding concentration, and Bottom and Top represent the minimum and maximum inhibition percentage, respectively.

Experimental results: IC50 values of the inhibitory effect of the test compounds on hERG potassium channel current are shown in Table 4.

Table 4 Inhibition of test compounds on hERG potassium channel current

| Test compound | Inhibition rate at the highest concentration tested | $IC_{50}$ (μM) |
|---|---|---|
| LX-9211 | 102.2 ± 4.08% at 40 μM | 1.82 |
| Compound 2 | 86.6 ± 1.77% at 40 μM | 8.75 |
| Compound 9 | 68.9 ± 1.80% at 40 μM | 24.1 |

**Example 5: Spinal nerve ligation (SNL)-induced mouse model of neuropathic pain**

Male C57BL/6J mice (8 weeks old) purchased from Jinan Pengyue Experimental Animal Breeding Co., Ltd. were adaptively raised for one week and then the models were established. The specific establishment method comprises the following steps:

1) disinfecting the surgical instrument and ligature;
2) anesthetizing the mice with isoflurane and then placing the mice on the operating table in the prone position;
3) shaving the fur near the hip bones of the mice and preparing the skin, and making an incision of about 2 cm along the spine;
4) isolating the fascia along the spine, performing blunt dissection of the muscles, and exposing the transverse process of L5;
5) carefully cutting the transverse process of L5 with forceps and exposing the L5 spinal nerve;
6) carefully isolating the L5 nerve with a glass dissecting needle, and ligating the L5 nerve with a 5-0 ligature; and

7) suturing the muscles and skin and disinfecting same with iodophor.

The mice that were unsuccessful in modelling were eliminated the next day after modelling (marker for successful modelling: mice with their hind paws curled up). After modelling, the mice were stroked for 3 to 5 minutes every day to ensure that the animals were familiar with the experimenter, and then the mice were placed on a metal pain measuring frame for 40 to 60 minutes of adaptation. After 3 days of acclimatization, Von Frey filaments (Aesthesio®; 0.16 g, 0.4 g, 0.6 g, 1.0 g, 1.4 g and 2.0 g) were used to test the pre-administration baseline values of the animals (Ascending testing approach). Each animal was tested twice and average values were taken, with intervals of at least 5 minutes. The animals were grouped according to the baseline values (10 animals per group). After the grouping, LX-9211 (1 and 10 mg/kg), compound 2 (1 and 10 mg/kg) or vehicle (40% PEG-400 + 10% ethanol + 15% Tween 80 + 35% physiological saline) was administered intragastrically. The mice were tested for the mechanical pain threshold (MPT) at 1, 3 and 6 hours after the administration. Time-MPT curve was plotted using GraphPad 8.3.0, and statistical analysis was performed.

Results and conclusion: the results are shown in Figure 1. At 1, 3 and 6 hours after a single administration, LX-9211 and compound 2 (both at the dose of 10 mg/kg) effectively increased the pain threshold of mice after SNL modelling. The analgesic efficacy of LX-9211 (10 mg/kg) reached the peak at 1 hour after the administration, and the efficacy gradually decreased thereafter. The analgesic efficacy of compound 2 (10 mg/kg) reached the peak at 3 hours after the administration and tended to be stable at 1 hour to 6 hours after the administration. Compound 2 had better efficacy than LX-9211 at 3 and 6 hours after the administration. The above data show that as an analgesic, compound 2 has better pharmacodynamic activity than LX-9211.

**Example 6: Mouse brain/blood ratio test**

6.1 Experimental animals: male ICR mice, 20 to 25 g, 9 mice/compound. purchased from Chengdu Ddossy Experimental Animals Co., Ltd.

6.2 Experimental design: on the day of the experiment, 18 ICR mice were randomly grouped according to their body weights. The animals were fasted with water available for 12 to 14 h one day before the administration and were fed 4 h after the administration.

Table 5. Administration information

| Group | Number | Administration information | | | | | |
|---|---|---|---|---|---|---|---|
| | Male | Test compound | Administration dosage (mg/kg) | Administration concentration (mg/mL) | Administration volume (mL/kg) | Collected sample | Mode of administration |
| G1 | 9 | LX9211 | 10 | 1 | 10 | Plasma | Intragastric administration |
| G2 | 9 | Compound 2 | 10 | 1 | 10 | Plasma | Intragastric administration |
| Note: vehicle for intragastric administration: 40% PEG-400 + 10% Ethanol + 15% Tween 80 + 35% Saline; (Saline; Ethanol; Tween 80) | | | | | | | |

After intragastric administration, whole blood and brain tissue were collected at 0.5 h, 4 h and 24 h, and the whole blood was centrifuged to separate the plasma. The brain tissue was rinsed with cold physiological saline to remove the residual blood on the surface, drained out and then homogenized. Before analysis and detection, all samples were stored at -80°C, and the samples were quantitatively analysed by LC-MS/MS.

The test results are shown in Table 6.

Table 6. Pharmacokinetic parameters of compounds in plasma of mice

| Test compound | Mode of administration | Plasma $AUC_{0-t}$ (hr*ng/mL) | Brain tissue $AUC_{0-t}$ (hr*ng/g) | Brain/plasma ratio |
|---|---|---|---|---|
| LX9211 | i.g. (10 mg/kg) | 6643 | 110424 | 16.6 |
| Compound 2 | i.g. (10 mg/kg) | 572 | 36853 | 64.5 |
| -: not applicable. | | | | |

Conclusion: the compounds of the present invention, especially compound 2, have a high brain penetrability.

**Example 7: Compositions of compound 3**

7.1 Formulations 1 to 6

Table 7. Formulations of AAK1 inhibitor-containing compositions (1)

| **Name of raw materials** | **Function** | **Amount (%)** | | | | | |
|---|---|---|---|---|---|---|---|
| | | **Formulat ion 1** | **Formulat ion 2** | **Formulat ion 3** | **Formulat ion 4** | **Formulat ion 5** | **Formulat ion 6** |
| Compound 3 | Active drug | 10 | 10 | 10 | 10 | 10 | 10 |
| Silica | Infiltrating agent | / | 2 | 5 | 10 | 10 | 10 |
| Talc | | / | / | / | / | 5 | 10 |
| Microcrystal line | Diluent | 60 | 58 | 55 | 50 | 45 | 40 |
| cellulose | | | | | | | |
| Lactose | | 24.5 | 24.5 | 24.5 | 24.5 | 24.5 | 24.5 |
| Low-substituted hydroxypropyl cellulose | Disintegrant | 5 | 5 | 5 | 5 | 5 | 5 |
| Magnesium stearate | Lubricant | 0.5 | 0.5 | 0.5 | 0.5 | 0.5 | 0.5 |

Tablets containing compositions of compound 3 were prepared according to the formulations in the table: first, compound 3 and an infiltrating agent were cogrinded and mixed for 15 minutes, then a diluent and a disintegrant were added and mixed in a mixer for 15 minutes, and finally a lubricant was added and mixed for 3 minutes to obtain compositions containing compound 3; the compositions were compressed into tablets using a tablet press.

We observed whether, during the process, there were phenomena such as agglomeration in the composition or the composition adhering to the equipment resulting from the softening or melting of compound 3 in the above-mentioned formulations. In addition, we investigated the content uniformity of the finished product.

Table 8. Experimental results

| **Phenomenon and result** | **Formula tion 1** | **Formulat ion 2** | **Formulati on 3** | **Formulati on 4** | **Formulati on 5** | **Formulati on 6** |
|---|---|---|---|---|---|---|
| Is there agglomeration in the composition? | No | No | No | No | No | No |
| Is there adhesion to equipment? | No | No | No | No | No | No |
| Content uniformity (A+2.2S) | 4.3 | 3.8 | 5.2 | 4.6 | 4.3 | 5.5 |

The results indicate that, regardless of whether an infiltrating agent is added to the formulation, no phenomena of agglomeration or adhesion to equipment resulting from the softening or melting of compound 3 have occurred.

7.2 Formulations 7 to 12

Table 9. Formulations of AAK1 inhibitor-containing compositions (2)

| **Name of raw materials** | **Function** | **Amount (%)** | | | | | |
|---|---|---|---|---|---|---|---|
| | | **Formulation 7** | **Formulation 8** | **Formulation 9** | **Formulation 10** | **Formulation 11** | **Formulation 12** |
| Compound 3 | Active drug | 20 | 40 | 80 | 40 | 40 | 40 |
| Silica | Infiltrating agent | / | / | / | 3 | 6 | 10 |
| Magnesium silicate | Infiltrating agent | / | / | / | 2 | 4 | 5 |
| Microcrystalline cellulose | Diluent | 45 | 35 | 10 | 30 | 25 | 20 |
| Mannitol | Diluent | 29.5 | 19.5 | 4.5 | 19.5 | 19.5 | 19.5 |
| Crospovidone | Disintegrant | 5 | 5 | 5 | 5 | 5 | 5 |
| Magnesium stearate | Lubricant | 0.5 | 0.5 | 0.5 | 0.5 | 0.5 | 0.5 |

Tablets containing compositions of compound 3 were prepared according to the formulations in the table: first, compound 3 and an infiltrating agent were sieved and mixed 5 times, then mixed in a mixer for 15 minutes, and then a diluent and a disintegrant were added and mixed for 15 minutes, and finally a lubricant was added and mixed for 3 minutes to obtain compositions containing compound 3; the compositions were compressed into tablets using a tablet press.

We observed whether, during the process, there were phenomena such as agglomeration in the composition or the composition adhering to the equipment resulting from the softening or melting of compound 3 in the above-mentioned formulations. **In** addition, we investigated the content uniformity of the finished product.

Table 10. Experimental results

| **Phenomenon and result** | **Formulation 7** | **Formulation 8** | **Formulation 9** | **Formulation 10** | **Formulation 11** | **Formulation 12** |
|---|---|---|---|---|---|---|
| Is there agglomeration in the composition? | / | Yes | Yes | No | No | No |
| Is there adhesion to equipment? | / | Yes | Yes | / | No | No |
| Content uniformity (A+2.2S) | / | 10.5 | 13.2 | 3.6 | 4.6 | 3.5 |

Conclusion: the addition of an appropriate amount of an infiltrating agent to the formulation can significantly mitigate the softening/melting phenomenon of compound 3 during the preparation process, and as a result, the tablets exhibit better content uniformity.

7.3 Formulations 13 to 18

Table 11. Formulations of AAK1 inhibitor-containing compositions (3)

| Name of raw materials | Function | Amount (%) | | | | | |
|---|---|---|---|---|---|---|---|
| | | Formulation 13 | Formulation 14 | Formulation 15 | Formulation 16 | Formulation 17 | Formulation 18 |
| Compound 3 | Active drug | 20 | 40 | 80 | 40 | 40 | 40 |
| Silica | Infiltrating agent | / | / | / | 5 | 10 | 15 |
| Pregelatiniz ed starch | Diluent | 40 | 30 | 10 | 30 | 25 | 20 |
| Lactose | | 35 | 25 | 5 | 20 | 20 | 20 |
| Sodium carboxymet hyl starch | Disintegr ant | 3 | 3 | 3 | 3 | 3 | 3 |
| Talc | Lubricant | 2 | 2 | 2 | 2 | 2 | 2 |

Preparation method: compound 3 and an infiltrating agent were mixed in a mixer for 15 minutes, and then a diluent and a disintegrant were added and mixed for 15 minutes, and finally magnesium stearate was added and mixed for 3 minutes to obtain compositions containing compound 3; the compositions containing compound 3 were filled into capsules using a capsule filling machine.

We observed whether, during the process, there were phenomena such as agglomeration in the composition or the composition adhering to the equipment resulting from the softening or melting of compound 3 in the above-mentioned formulations. **In** addition, we investigated the content uniformity of the finished product.

Table 12. Experimental results

| **Phenomenon and result** | **Formulation 13** | **Formulation 14** | **Formulation 15** | **Formulation 16** | **Formulation 17** | **Formulation 18** |
|---|---|---|---|---|---|---|
| Is there agglomeration in the composition? | No | Yes | Yes | No | No | No |
| Is there adhesion to equipment? | No | Yes | Yes | No | No | No |
| Content uniformity (A+2.2S) | 4.6 | 8.3 | 11.0 | 4.1 | 5.5 | 3.9 |

Conclusion: the addition of an infiltrating agent can significantly inhibit the softening/melting phenomenon of compound 3 during the preparation process.

7.4 Formulations 19 to 24

Table 13. Formulations of AAK1 inhibitor-containing compositions (4)

| Name of raw materials | Function | Amount (%) | | | | | |
|---|---|---|---|---|---|---|---|
| | | Formulation 19 | Formulation 20 | Formulation 21 | Formulation 22 | Formulation 23 | Formulation 24 |
| Compound 3 | Active drug | 20 | 40 | 80 | 40 | 40 | 40 |
| Silica | Infiltrating agent | / | / | / | 5 | 10 | 15 |
| Microcrystalline cellulose | Diluent | 45 | 35 | 10 | 30 | 25 | 20 |
| Lactose | | 29.5 | 19.5 | 4.5 | 19.5 | 19.5 | 19.5 |
| Croscarmellose sodium | Disintegrant | 5 | 5 | 5 | 5 | 5 | 5 |
| Magnesium stearate | Lubricant | 0.5 | 0.5 | 0.5 | 0.5 | 0.5 | 0.5 |

Tablets containing compositions of compound 3 were prepared according to the formulations in the table: first, compound 3 and an infiltrating agent were sieved and mixed 5 times, then mixed in a mixer for 15 minutes, and then a diluent and a disintegrant were added and mixed for 15 minutes, and subsequently the premix was fed into a roller compactor and compacted at 0.6 kN/cm; the resultant ribbon-like material was passed through a 0.8 mm vibration mill; the milled particles were then mixed with a lubricant for 3 minutes, and finally the mixture was compressed into tablets using a tablet press.

We investigated the dissolution behaviour of the tablets prepared according to the above-mentioned formulations and processes in a pH 1.0 medium, as well as the changes in their related substances when stored at 40°C ± 2°C, 75% RH ± 5% RH for 6 months, see Table 14.

As can be seen from Figure 2, for formulations 19 to 21, with the increase in the proportion of compound 3, the agglomeration of AAK1 resulting from its softening/melting further decreases its dissolution rate. For formulations 22 to 24, the addition of an infiltrating agent to the formulations can avoid the agglomeration resulting from the softening/melting of compound 3, thereby improving its dissolution behaviour.

Table 14. Stability results

| **Storage time** | **Total impurity (%)** | | | | | |
|---|---|---|---|---|---|---|
| | **Formulation 19** | **Formulation 20** | **Formulation 21** | **Formulation 22** | **Formulation 23** | **Formulation 24** |
| 0 days | 0.584 | 0.562 | 0.581 | 0.576 | 0.543 | 0.572 |
| 1 month | 0.607 | 0.557 | 0.605 | 0.586 | 0.552 | 0.565 |
| 2 months | 0.575 | 0.586 | 0.615 | 0.602 | 0.548 | 0.573 |
| 3 months | 0.600 | 0.598 | 0.624 | 0.615 | 0.566 | 0.598 |
| 6 months | 0.621 | 0.625 | 0.648 | 0.636 | 0.594 | 0.615 |

As can be seen from the table, regardless of whether the composition contains an infiltrating agent, it demonstrates good stability.

7.5 Formulations 25 to 28

Table 15. Formulations of AAK1 inhibitor-containing compositions (5)

| **Name of raw materials** | **Function** | **Amount (mg)** |
|---|---|---|
| | | **Formulation 25** |
| Compound 3 | Active drug | 10 |
| Microcrystalline cellulose | Diluent | 62 |
| Lactose | | 22 |
| Croscarmellose sodium | Disintegrant | 5 |
| Magnesium stearate | Lubricant | 1 |

Preparation method: compound 3 was mixed with lactose, microcrystalline cellulose and croscarmellose sodium in a mixer for 15 minutes, and then magnesium stearate was added and mixed for 3 minutes; the mixture was then compressed into tablets using a tablet press, with the tablet hardness controlled within the range of 50 to 100 N.

Table 16. Formulations of AAK1 inhibitor-containing compositions (6)

| **Name of raw materials** | **Function** | **Amount (mg)** |
|---|---|---|
| | | **Formulation** 26 |
| Compound 3 | Active drug | 25 |

(continued)

| Name of raw materials | Function | Amount (mg) |
|---|---|---|
| | | **Formulation** 26 |
| Microcrystalline cellulose | Diluent | 40 |
| Mannitol | | 30 |
| Low-substituted carboxymethyl cellulose sodium | Disintegrant | 3 |
| Talc | Lubricant | 2 |

Preparation method: compound 3 was mixed with mannitol, microcrystalline cellulose and low-substituted carboxymethyl cellulose sodium in a premixer for 15 minutes, and then the premix was fed into a roller compactor and compacted at 0.6 kN/cm; the resultant ribbon-like material was passed through a 0.8 mm vibration mill; the milled particles were then mixed with talc for 3 minutes, and finally the mixture was compressed into tablets using a tablet press, with the tablet hardness controlled within the range of 50 to 100 N.

Table 17. Formulations of AAK1 inhibitor-containing compositions (7)

| Name of raw materials | Function | Amount (mg) |
|---|---|---|
| | | **Formulation** 27 |
| Compound 3 | Active drug | 50 |
| Pregelatinized starch | Diluent | 75 |
| Dextrin | | 70 |
| Sodium carboxymethyl starch | Disintegrant | 3 |
| Magnesium stearate | Lubricant | 2 |

Preparation method: compound 3 was mixed with pregelatinized starch, sodium carboxymethyl starch and dextrin in a mixer for 15 minutes, and then magnesium stearate was added and mixed for 3 minutes; the mixture was then compressed into tablets using a tablet press, with the tablet hardness controlled within the range of 50 to 100 N.

Table 18. Formulations of AAK1 inhibitor-containing compositions (8)

| Name of raw materials | Function | Amount (mg) |
|---|---|---|
| | | **Formulation** 28 |
| Compound 3 | Active drug | 100 |
| Calcium phosphate | Diluent | 80 |
| Sucrose | | 115 |
| Crospovidone | Disintegrant | 3 |
| Talc | Lubricant | 2 |

Preparation method: compound 3 was mixed with calcium phosphate, sucrose, crospovidone and talc in a mixer for 15 minutes, and then the mixture was filled into capsules using a capsule filling machine.

In accordance with Method 2 (Paddle Method) in the Dissolution and Release Test (section 0931) of the 2015 edition of the Chinese Pharmacopoeia, the dissolution curves of the tablets prepared according to formulation 25 in water, a pH 1.0 medium, a pH 4.5 medium, and a pH 6.8 medium were investigated. The rotation speed was set to 50 rpm, and the sampling time points were 5 min, 10 min, 15 min, 20 min, 30 min, 45 min, and 60 min.

As can be seen from the results in Figure 3, when pH = 1.0, the dissolution rate of the tablets prepared according to formulation 25 can reach 90% within 5 minutes, indicating rapid dissolution; at 20 minutes, the dissolution rate of the tablets prepared according to formulation 25 in water, a pH 1.0 medium, a pH 4.5 medium, and a pH 6.8 medium can all reach 85%.

### 7.6 Study methods and results of formulation stability

The formulations prepared according to formulation 25, formulation 26, formulation 27 and formulation 28 were internally packaged in the form of aluminium-plastic blister packages, stored at 40°C ± 2°C, 75% RH ± 5% RH, and then investigated for the changes in their related substances.

Table 19. Stability investigation results

| Storage time | Total impurity (%) | | | |
|---|---|---|---|---|
| | Formulation 25 | Formulation 26 | Formulation 27 | Formulation 28 |
| 0 days | 0.584 | 0.562 | 0.581 | 0.576 |
| 1 month | 0.607 | 0.557 | 0.605 | 0.586 |
| 2 months | 0.575 | 0.586 | 0.615 | 0.602 |
| 3 months | 0.600 | 0.598 | 0.624 | 0.615 |
| 6 months | 0.621 | 0.625 | 0.648 | 0.636 |

As can be seen from the results in Table 19, the formulations prepared according to formulations 25-28 all exhibit relatively high stability, and their stabilities are essentially equivalent.

The pharmaceutical compositions of compound 2 were prepared by referencing formulations 1-6, 10-13 and 16-28 and processes of the pharmaceutical compositions of compound 3. No phenomena of agglomeration or adhesion to equipment were observed. The content uniformity (A+2.2S) ranged from 3 to 5.5, and all the pharmaceutical compositions demonstrated relatively high stability.

## Claims

1. A pharmaceutical composition, **characterized by** comprising:

an active ingredient, wherein the active ingredient is selected from a compound of formula (I), or a stereoisomer or a pharmaceutically acceptable salt thereof:

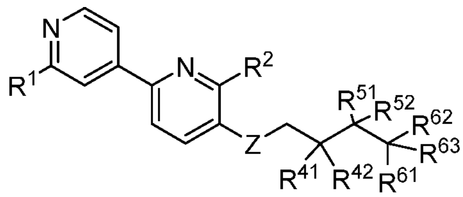

(I)

wherein
Z is selected from NH or O;
$R^1$ and $R^2$ are each independently selected from H, deuterium, halogen, amino, -COOH, cyano, sulfonyl, aminoacyl, $C_{1-6}$ alkyl, halo $C_{1-6}$ alkyl, or deuterated $C_{1-6}$ alkyl, wherein the alkyl is optionally further substituted with 1-3 $R^A$ substituents;
$R^{41}$ and $R^{42}$ are each independently selected from H, deuterium, amino, $C_{1-6}$ alkyl, halogen, cyano, hydroxyl, halo $C_{1-6}$ alkyl, or deuterated $C_{1-6}$ alkyl;
$R^{51}$ and $R^{52}$ are each independently selected from H, deuterium, amino, or halogen;
$R^{61}$, $R^{62}$ and $R^{63}$ are each independently selected from H, deuterium, halogen, amino, cyano, hydroxyl, $C_{1-6}$ alkyl, halo $C_{1-6}$ alkyl, or deuterated $C_{1-6}$ alkyl;
or, $R^{51}$ and $R^{61}$, or $R^{61}$ and $R^{62}$ together with the carbon atom(s) to which they are each attached form a double bond;
$R^A$ is selected from deuterium, halogen, amino, cyano, hydroxyl, $C_{1-6}$ alkyl, halo $C_{1-6}$ alkyl, deuterated $C_{1-6}$ alkyl, $C_{1-6}$ alkoxy, halo $C_{1-6}$ alkoxy, deuterated $C_{1-6}$ alkoxy, or hydroxy $C_{1-6}$ alkyl,
provided that when Z is selected from O,

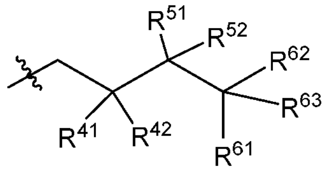

does not form the following structures:

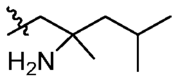 and 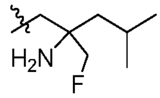

b) a non-active ingredient;
wherein the active ingredient is present in the pharmaceutical composition in an amount of 5% to 90% w/w, preferably 5% to 80% w/w, and more preferably 10% to 80% w/w.

2. The pharmaceutical composition according to claim 1, **characterized in that**

Z is O;
R1 and R2 are selected from halo $C_{1-2}$ alkyl;
R51 and R52 are each independently selected from H or deuterium;
R41 and R42 are each independently selected from amino or C1-2 alkyl;
R61, R62 and R63 are each independently selected from H, deuterium, or C1-2 alkyl.

3. The pharmaceutical composition according to claim 1, **characterized in that** the compound of formula (I) is selected from one of the following structures:

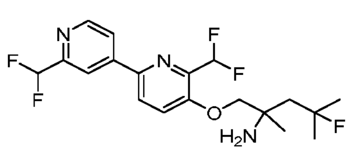 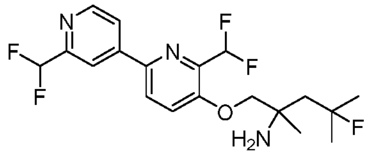 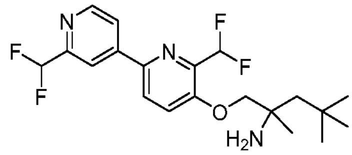

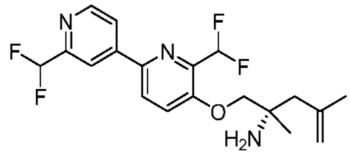 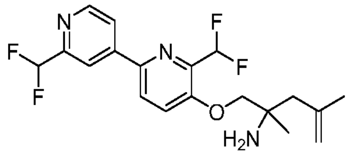 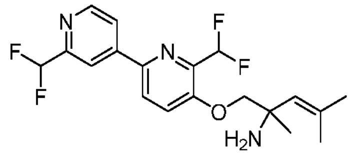

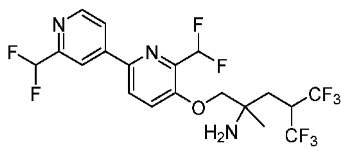 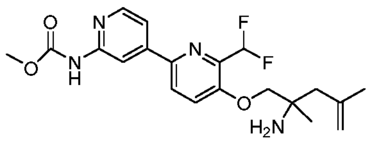 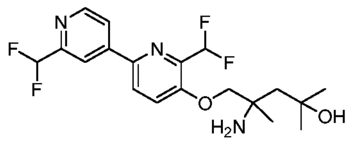

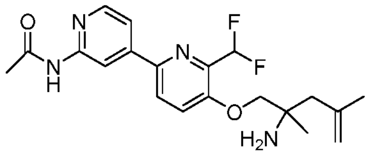 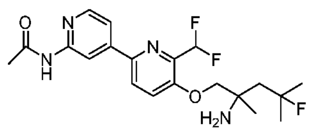 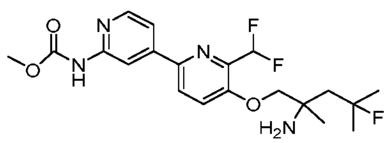

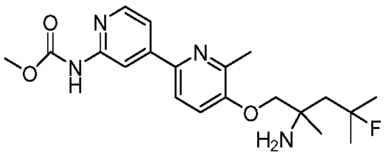 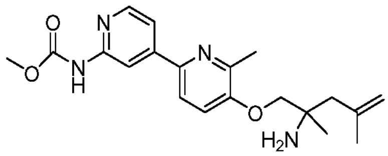

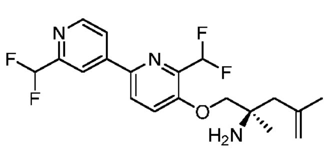 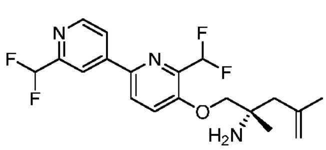 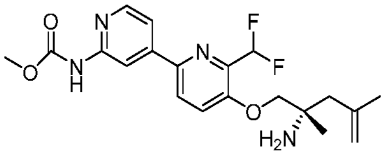

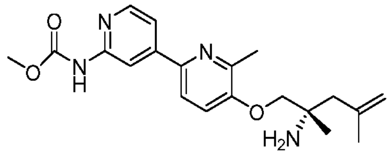 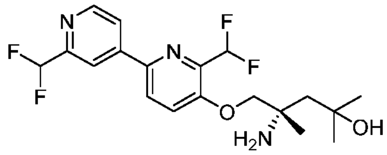

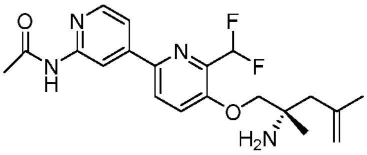 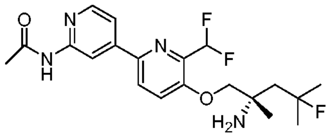 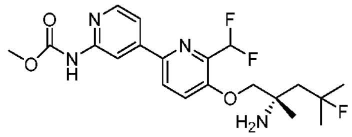

or

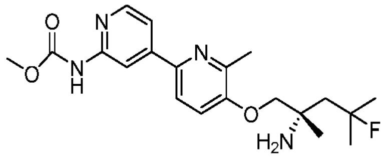.

4. The pharmaceutical composition according to claim 1, **characterized in that** the non-active ingredient is selected from a diluent.

5. The pharmaceutical composition according to claim 4, **characterized in that** the diluent is selected from one or more of starch, pregelatinized starch, dextrin, lactose monohydrate, anhydrous lactose, sucrose, microcrystalline cellulose, inorganic salts, and sugar alcohols; preferably, the diluent is one or more of pregelatinized starch, dextrin, lactose, sucrose, microcrystalline cellulose, calcium sulphate, calcium hydrogenphosphate dihydrate, anhydrous calcium hydrogenphosphate, calcium phosphate, calcium carbonate, calcium stearate, magnesium oxide, aluminium hydroxide, mannitol, xylitol, and sorbitol; more preferably, the diluent is one or more of pregelatinized starch, lactose, sucrose, microcrystalline cellulose, calcium hydrogenphosphate dihydrate, anhydrous calcium hydrogenphosphate, calcium phosphate, mannitol, and dextrin.

6. The pharmaceutical composition according to claim 4, **characterized in that** the weight ratio of the active ingredient to the diluent is 1:0.05 to 1:10, preferably 1:0.1 to 1:8.5, and further preferably 1:0.18 to 1:8.5.

7. The pharmaceutical composition according to claim 4, **characterized in that** the non-active ingredient further contains one or more of an infiltrating agent, a disintegrant, and a lubricant;

the infiltrating agent is selected from one or more of silicates; preferably, the infiltrating agent is one or more of silica, magnesium silicate, magnesium trisilicate, magnesium aluminium silicate and talc; more preferably, the infiltrating agent is one or more of fumed silica, precipitated silica, sol-gel silica, magnesium silicate, magnesium trisilicate, magnesium aluminium silicate and talc;

the disintegrant is selected from one or more of croscarmellose sodium, crospovidone, starch and a derivative thereof, low-substituted hydroxypropyl cellulose, low-substituted sodium hydroxymethyl cellulose, a surfactant, alginic acid and sodium alginate, and clays; preferably, the disintegrant is one or more of croscarmellose sodium, low-substituted hydroxypropyl cellulose, low-substituted sodium hydroxymethyl cellulose, crospovidone, starch, sodium carboxymethyl starch, hydroxypropyl starch, polysorbate 80, sodium lauryl sulphate, bentonite, and colloidal magnesium aluminium silicate; more preferably, the disintegrant is one or more of croscarmellose sodium, low-substituted hydroxypropyl cellulose, low-substituted sodium hydroxymethyl cellulose, sodium carboxymethyl starch, and crospovidone;

the lubricant is selected from one or more of talc, stearic acid, metal stearate, stearate, glyceryl behenate, sodium lauryl sulphate or colloidal silica; preferably, the lubricant is one or more of talc, calcium stearate, magnesium stearate and zinc stearate, polyoxyethylene stearate, glyceryl monostearate, and glyceryl palmitostearate; more preferably, the lubricant is one or more of talc, calcium stearate, magnesium stearate, and glyceryl monostearate.

8. The pharmaceutical composition according to claim 7, **characterized in that** the weight ratio of the active ingredient to the infiltrating agent is 1:0.05 to 1:5,

   preferably 1:0.08 to 1:3, and further preferably 1:0.12 to 1:2;
   the weight ratio of the active ingredient to the disintegrant is 1:0.01 to 1:3, preferably 1:0.03 to 1:1.5, and further preferably 1:0.03 to 1:0.6;
   the weight ratio of the active ingredient to the lubricant is 1:0.001 to 1:2, preferably 1:0.006 to 1:0.1.

9. A pharmaceutical formulation, **characterized by** comprising the pharmaceutical composition according to any one of claims 1 to 8.

10. The pharmaceutical formulation according to claim 9, **characterized in that** the amount of the active ingredient in a unit formulation is from 1 mg to 100 mg, preferably 5 mg, 10 mg, 20 mg, 25 mg, 30 mg, 40 mg, 50 mg, 60 mg, 70 mg, 80 mg, 90 mg, or 100 mg.

11. The pharmaceutical formulation according to claim 9, **characterized in that** the formulation form of the pharmaceutical formulation is selected from a tablet, a granule, a capsule, or a soft capsule.

12. Use of the pharmaceutical composition according to any one of claims 1 to 8 or the pharmaceutical formulation according to any one of claims 9 to 11 in the preparation of a medicament for treating an AAK1-related disease by inhibition or degradation of AAK1.

13. The use according to claim 12, **characterized in that** the disease is selected from pain, including inflammatory pain, postoperative pain, trigeminal neuralgia, acute postherpetic neuralgia and postherpetic neuralgia, diabetic peripheral neuralgia, causalgia, occipital neuralgia, fibromyalgia, phantom limb pain, burn pain and other forms of neuralgia, neuropathy and spontaneous pain syndrome.

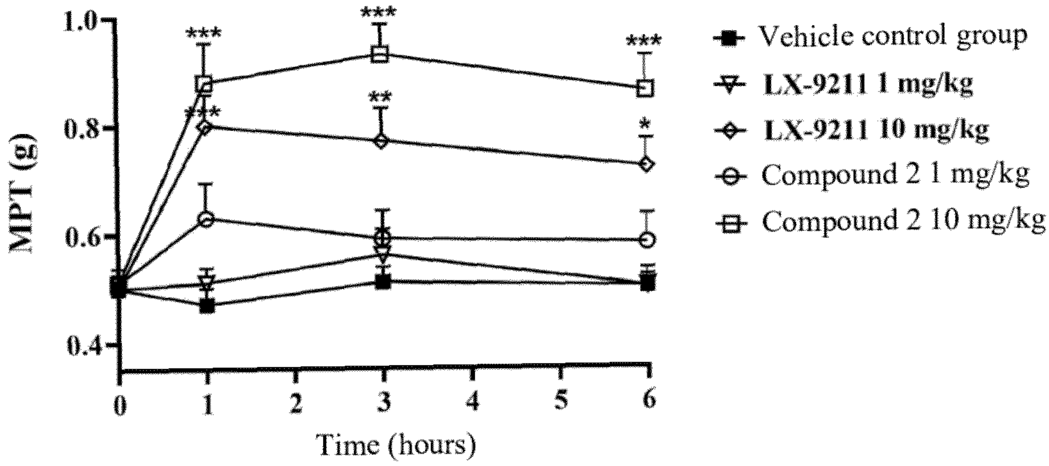
1.0
0.8
0.6
0.4
MPT (g)
***
***
***
***
**
*
0
1
2
3
4
5
6
Time (hours)
Vehicle control group
LX-9211 1 mg/kg
LX-9211 10 mg/kg
Compound 2 1 mg/kg
Compound 2 10 mg/kg

FIG. 1

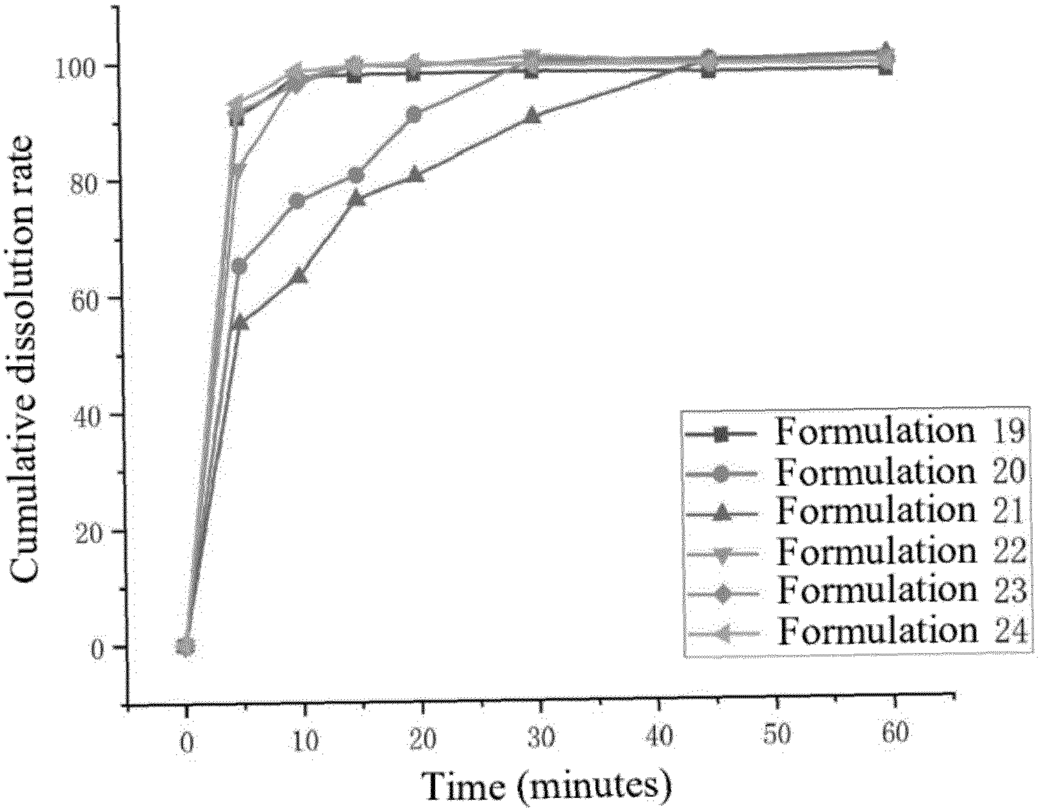
100
80
60
40
20
0
Cumulative dissolution rate
0
10
20
30
40
50
60
Time (minutes)
Formulation 19
Formulation 20
Formulation 21
Formulation 22
Formulation 23
Formulation 24

FIG. 2

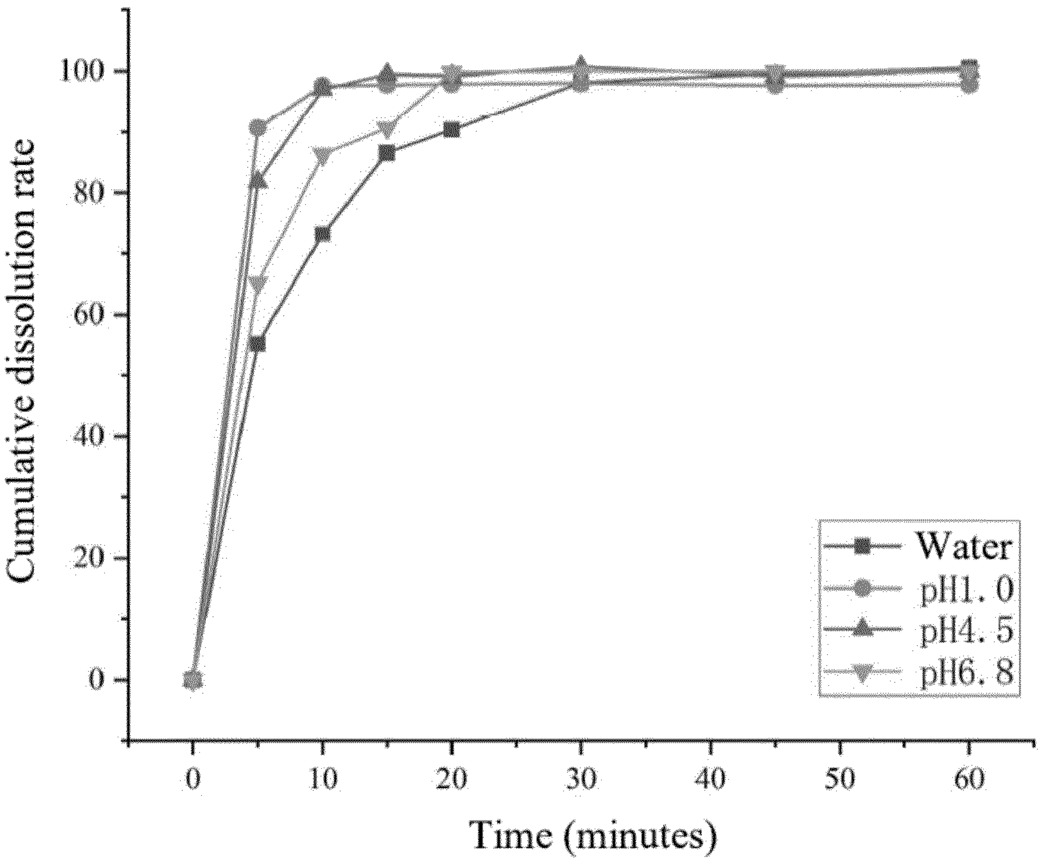
Cumulative dissolution rate
Time (minutes)
0
20
40
60
80
100
0
10
20
30
40
50
60
Water
pH1. 0
pH4. 5
pH6. 8

FIG. 3

## INTERNATIONAL SEARCH REPORT

International application No.
**PCT/CN2023/130976**

**A. CLASSIFICATION OF SUBJECT MATTER**

A61K31/444(2006.01)i; C07D401/04(2006.01)i; A61P25/00(2006.01)i

According to International Patent Classification (IPC) or to both national classification and IPC

**B. FIELDS SEARCHED**

Minimum documentation searched (classification system followed by classification symbols)

IPC:A61K C07D A61P

Documentation searched other than minimum documentation to the extent that such documents are included in the fields searched

Electronic data base consulted during the international search (name of data base and, where practicable, search terms used)

CNTXT; WPABSC; ENTXTC; ENTXT; CJFD; STN; Bing; CNKI: 二甲基戊烷 5w (胺 or 烯 or 氟), 联吡啶, AAK1, AP-2相关激酶1, 疼, 痛, neuropathic pain, NP, hurt, pain, ache, AP2 associated kinase 1, 海思科, HAISCO, STN structural formula search

**C. DOCUMENTS CONSIDERED TO BE RELEVANT**

| Category* | Citation of document, with indication, where appropriate, of the relevant passages | Relevant to claim No. |
|---|---|---|
| X | CN 108368084 A (BRISTOL MYERS SQUIBB COMPANY) 03 August 2018 (2018-08-03) description, paragraphs [0007]-[0030], [0052], [0054], [0069], [0074]-[0075] and [0085]-[0090], and embodiment 273, and claims 4-7 | 1-13 |
| X | CN 108290843 A (BRISTOL MYERS SQUIBB COMPANY) 17 July 2018 (2018-07-17) claims 6 and 10-12, and description, paragraphs [0006]-[0033], [0037], [0056]-[0057] and [0069]-[0074], and embodiment 277 | 1-13 |
| X | CN 106458994 A (BRISTOL MYERS SQUIBB COMPANY) 22 February 2017 (2017-02-22) claims 6 and 11-15, and description, paragraphs [0007]-[0043], [0062]-[0065] and [0077]-[0080], and embodiments 63-67, 117, 119, 123, 161 and 179 | 1-13 |
| X | WO 2021216441 A1 (LEXICON PHARMACEUTICALS, INC.) 28 October 2021 (2021-10-28) claims 13-27, and description, page 1, lines 25-27, and page 27, line 16 to page 29, line 9 | 1-13 |
| PX | WO 2023284838 A1 (SICHUAN HAISCO PHARMACEUTICAL CO., LTD.) 19 January 2023 (2023-01-19) claims 9 and 10-15, and description, paragraphs [0005], [0129] and [0172] | 1-13 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **15 December 2023** | **22 December 2023** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**

International application No.
**PCT/CN2023/130976**

**C. DOCUMENTS CONSIDERED TO BE RELEVANT**

| Category* | Citation of document, with indication, where appropriate, of the relevant passages | Relevant to claim No. |
|---|---|---|
| A | US 2005085642 A1 (SANKIO CHEMICAL CO., LTD.) 21 April 2005 (2005-04-21)<br>claims 1-3, and description, paragraphs [0048]-[0050] | 1-13 |
| A | WO 2021216454 A1 (LEXICON PHARMACEUTICALS, INC.) 28 October 2021 (2021-10-28)<br>claims 1-64 | 1-13 |
| A | PANG, Jiahao et al.,. "Revisiting the Chichibabin reaction: C2-amination of pyridines with a NaH-iodide composite"<br>*ASIAN JOURNAL OF ORGANIC CHEMISTRY,*<br>Vol. 8, No. 7, 31 December 2019 (2019-12-31), pages 1-4<br>page 3, left-hand column, paragraphs 1-2, route 5 | 1-13 |
| A | CN 105683196 A (BRISTOL MYERS SQUIBB COMPANY) 15 June 2016 (2016-06-15)<br>embodiments 17, 26 and 36, and description, paragraphs [292] and [306], and table 1a | 1-13 |

Form PCT/ISA/210 (second sheet) (July 2022)

**INTERNATIONAL SEARCH REPORT**
**Information on patent family members**

International application No.
**PCT/CN2023/130976**

| Patent document cited in search report | | | Publication date (day/month/year) | Patent family member(s) | | | Publication date (day/month/year) |
|---|---|---|---|---|---|---|---|
| CN | 108368084 | A | 03 August 2018 | EP | 3356340 | A1 | 08 August 2018 |
| | | | | EP | 3356340 | B1 | 23 October 2019 |
| | | | | ES | 2765730 | T3 | 10 June 2020 |
| | | | | KR | 20180056765 | A | 29 May 2018 |
| | | | | US | 2018346440 | A1 | 06 December 2018 |
| | | | | US | 10544120 | B2 | 28 January 2020 |
| | | | | WO | 2017059085 | A1 | 06 April 2017 |
| | | | | JP | 2018537408 | A | 20 December 2018 |
| | | | | JP | 6785302 | B2 | 18 November 2020 |
| CN | 108290843 | A | 17 July 2018 | US | 2019040080 | A1 | 07 February 2019 |
| | | | | US | 10246469 | B2 | 02 April 2019 |
| | | | | EP | 3356330 | A1 | 08 August 2018 |
| | | | | EP | 3356330 | B1 | 20 November 2019 |
| | | | | ES | 2767776 | T3 | 18 June 2020 |
| | | | | JP | 2018529731 | A | 11 October 2018 |
| | | | | JP | 6864674 | B2 | 28 April 2021 |
| | | | | KR | 20180056764 | A | 29 May 2018 |
| | | | | WO | 2017059080 | A1 | 06 April 2017 |
| CN | 106458994 | A | 22 February 2017 | AU | 2015240869 | A1 | 17 November 2016 |
| | | | | AU | 2015240869 | B2 | 08 November 2018 |
| | | | | CA | 2944466 | A1 | 08 October 2015 |
| | | | | CA | 2944466 | C | 09 June 2020 |
| | | | | HUE | 041457 | T2 | 28 May 2019 |
| | | | | EA | 201691916 | A1 | 28 February 2017 |
| | | | | EA | 028942 | B1 | 31 January 2018 |
| | | | | IL | 248086 | A0 | 30 November 2016 |
| | | | | IL | 248086 | B | 26 September 2019 |
| | | | | BR | 112016022298 | A2 | 15 August 2017 |
| | | | | BR | 112016022298 | B1 | 20 September 2022 |
| | | | | WO | 2015153720 | A1 | 08 October 2015 |
| | | | | CY | 1120988 | T1 | 11 December 2019 |
| | | | | ES | 2700549 | T3 | 18 February 2019 |
| | | | | PT | 3126351 | T | 04 December 2018 |
| | | | | NZ | 725814 | A | 25 June 2021 |
| | | | | SG | 11201608195 | QA | 28 October 2016 |
| | | | | US | 2019367508 | A1 | 05 December 2019 |
| | | | | US | 10723734 | B2 | 28 July 2020 |
| | | | | HRP | 20181899 | T1 | 11 January 2019 |
| | | | | DK | 3126351 | T3 | 14 January 2019 |
| | | | | PL | 3126351 | T3 | 29 March 2019 |
| | | | | US | 2018134704 | A1 | 17 May 2018 |
| | | | | US | 10155760 | B2 | 18 December 2018 |
| | | | | RS | 58062 | B1 | 28 February 2019 |
| | | | | US | 2017183340 | A1 | 29 June 2017 |
| | | | | US | 9902722 | B2 | 27 February 2018 |
| | | | | PE | 20170004 | A1 | 04 March 2017 |
| | | | | CL | 2016002502 | A1 | 23 June 2017 |
| | | | | SI | 3126351 | T1 | 30 November 2018 |
| | | | | MX | 2016012829 | A | 05 January 2017 |
| | | | | US | 2019048006 | A1 | 14 February 2019 |

Form PCT/ISA/210 (patent family annex) (July 2022)

**INTERNATIONAL SEARCH REPORT**
**Information on patent family members**

International application No.
**PCT/CN2023/130976**

| Patent document cited in search report | Publication date (day/month/year) | Patent family member(s) | Publication date (day/month/year) |
|---|---|---|---|
| | | US 10351563 B2 | 16 July 2019 |
| | | US 2021277001 A1 | 09 September 2021 |
| | | KR 20160132491 A | 18 November 2016 |
| | | KR 102379518 B1 | 25 March 2022 |
| | | EP 3126351 A1 | 08 February 2017 |
| | | EP 3126351 B1 | 19 September 2018 |
| | | LT 3126351 T | 26 November 2018 |
| | | ZA 201606767 B | 30 May 2018 |
| | | JP 2017511329 A | 20 April 2017 |
| | | JP 6411541 B2 | 24 October 2018 |
| | | US 2020317671 A1 | 08 October 2020 |
| | | US 10981910 B2 | 20 April 2021 |
| WO 2021216441 A1 | 28 October 2021 | EP 4138829 A1 | 01 March 2023 |
| | | US 2022054464 A1 | 24 February 2022 |
| | | US 11730723 B2 | 22 August 2023 |
| | | JP 2023522688 A | 31 May 2023 |
| | | AU 2021259419 A1 | 10 November 2022 |
| | | CA 3175970 A1 | 28 October 2021 |
| WO 2023284838 A1 | 19 January 2023 | TW 202317532 A | 01 May 2023 |
| US 2005085642 A1 | 21 April 2005 | JP 2005029517 A | 03 February 2005 |
| | | US 7301029 B2 | 27 November 2007 |
| WO 2021216454 A1 | 28 October 2021 | JP 2023522689 A | 31 May 2023 |
| | | AU 2021258128 A1 | 03 November 2022 |
| | | CA 3175973 A1 | 28 October 2021 |
| | | US 2022096481 A1 | 31 March 2022 |
| | | US 11666575 B2 | 06 June 2023 |
| | | EP 4138844 A1 | 01 March 2023 |
| CN 105683196 A | 15 June 2016 | JP 2016535759 A | 17 November 2016 |
| | | JP 6243539 B2 | 06 December 2017 |
| | | EA 201690244 A1 | 29 July 2016 |
| | | EA 029744 B1 | 31 May 2018 |
| | | EP 3044226 A1 | 20 July 2016 |
| | | EP 3044226 B1 | 06 September 2017 |
| | | US 2016222021 A1 | 04 August 2016 |
| | | MX 2016002924 A | 06 June 2016 |
| | | WO 2015038112 A1 | 19 March 2015 |
| | | CA 2941192 A1 | 19 March 2015 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- *CHEMICAL ABSTRACTS*, 1310584-14-5
- *CHEMICAL ABSTRACTS*, 564483-18-7